# EUROPÄISCHE PATENTSCHRIFT

(11) **EP 3 824 243 B1**
(45) Veröffentlichungstag und Bekanntmachung des Hinweises auf die Patenterteilung: **13.12.2023**
(21) Anmeldenummer: 19742164.7
(22) Anmeldetag: 15.07.2019
(51) Int. Cl.: F42B 3/11, F42B 3/107, F42B 3/198, A61N 1/375

(54) **METALL-FIXIERMATERIAL-DURCHFÜHRUNG MIT GERINGER FEHLERANFÄLLIGKEIT**
METAL FIXING MATERIAL LEADTHROUGH HAVING LOW SUSCEPTIBILITY TO FAULTS
PASSAGE EN MATÉRIAU DE FIXATION MÉTALLIQUE À FAIBLE RISQUE DE DÉFAILLANCE

(30) Priorität: 20.07.2018 DE 102018005733
(43) Veröffentlichungstag der Anmeldung: 26.05.2021
(60) Teilanmeldung: 21161856.6
(73) Patentinhaber: SCHOTT AG, 55122 Mainz (DE)
(72) Erfinder: PFEIFFER, Thomas, 84036 Kumhausen (DE); HARTL, Helmut, 1210 Wien (AT); RANFTL, Reinhard, 84076 Pfeffenhausen (DE); ROUSEK, Ondrej, 56401 Zamberk (CZ); NISHIWAKI, Susumu, Kokashi, Shiga 528-0042 (JP); HETTLER, Robert, 84036 Kumhausen (DE)
(74) Vertreter: Sawodny, Michael-Wolfgang
(86) Internationale Anmeldenummer: PCT/EP2019/068960
(87) Internationale Veröffentlichungsnummer: WO 2020/016153

(56) Entgegenhaltungen:
- EP-A1- 0 248 977
- EP-A2- 2 012 082
- DE-A1-102012 010 608
- DE-U1-202010 018 430

## Beschreibung

Die Erfindung betrifft eine Metall-Fixiermaterial-Durchführung, insbesondere für Einrichtungen, die hohen Drücken aussetzbar sind, bevorzugt Personenschutzeinrichtungen wie Anzünder von Airbags oder Gurtspannern, mit wenigstens einem Metallstift, der in ein Fixiermaterial, bevorzugt ein Glas- oder Glaskeramikmaterial eingeschmolzen ist.

Metall-Fixiermaterial-Durchführungen sind in verschiedenen Ausführungen aus dem Stand der Technik vorbekannt.

Metall-Fixiermaterial-Durchführungen umfassen eine vakuumdichte Verschmelzungen von Fixiermaterialien, insbesondere Gläsern, Glaskeramiken oder Kunststoffen mit Metallen. Die Metalle fungieren dabei als elektrische Leiter.

Stellvertretend wird dabei auf die US 5,345,872 A, oder die
US 3,274,937 A verwiesen. Derartige Durchführungen sind in der Elektronik und in der Elektrotechnik weit verbreitet. Das zum Einschmelzen verwendete Material, insbesondere Glas, dient hierbei als Isolator. Typische Metall-Fixiermaterial-Durchführungen sind derart aufgebaut, dass metallische Innenleiter in ein Glasmaterial eingebracht werden, wobei das Glasmaterial in ein äußeres Metallteil dem sogenannten Grundkörper, der aus einem ring- oder plattenförmigen Element gebildet wird, eingeschmolzen wird.

Als bevorzugte Anwendungen derartiger Metall-Fixiermaterial-Durchführungen gelten beispielsweise Zündeinrichtungen. Diese werden unter anderem für Airbags oder Gurtspanner bei Kraftfahrzeugen verwendet. In diesem Fall sind die Metall-Fixiermaterial-Durchführungen Bestandteil der Zündeinrichtung. Die gesamte Zündeinrichtung umfasst außer der Metall-Fixiermaterial-Durchführung eine Zündbrücke, den Sprengstoff sowie eine Metallabdeckung, die den Zündmechanismus dicht umschließt. Durch die Durchführung können entweder ein oder zwei oder mehr als zwei metallische Stifte hindurchgeführt werden. Bei einer besonders bevorzugten Ausführungsform mit einem metallischen Stift liegt das Gehäuse auf Masse, bei einer bevorzugten zweipoligen Ausführung die Masse auf einer der Stifte.

Aus der US 2006/0222881 A1, der US 2004/0216631 A, der EP 1 455 160 A, der US 2007/0187934 A1 sowie der US 1 813 906 A sind Metall-Fixiermaterial-Durchführungen insbesondere für Anzünder von Airbags oder Gurtspannern bekannt geworden, die sich dadurch auszeichnen, dass die Durchgangsöffnung für die Metallstifte aus dem Grundkörper ausgestanzt ist. Bei der Herstellung der Grundkörper werden gemäß der US 2007/0187934 A1 aus einem Bandmaterial mit einer Dicke im Bereich zwischen 1 mm und 5 mm, bevorzugt 1,5 mm und 3,5 mm, insbesondere zwischen 1,8 mm bis 3,0 mm, ganz besonders bevorzugt zwischen 2,0 mm bis 2,6 mm, die Öffnungen durch die gesamte Dicke des Grundkörpers D mittels des Stanzprozesses hindurchgetrieben.

Der Grundkörper wird allgemein auch Header genannt.

Bei der aus der WO 2012/110 242 A1 bekannt gewordenen Durchführung wird ein Grundkörper, in den ein Leiter eingeglast ist, in ein Gehäuse hermetisch dicht durch Schweißen, Löten, Einpressen, Einbördeln oder Einschrumpfen eingebracht. Das Gehäuseteil und/oder der Grundkörper, bevorzugt der im Wesentlichen ringförmige Grundkörper, umfassen in der WO 2012/ 110 242 A1 als Material ein Metall, insbesondere ein Leichtmetall, wie Titan, eine Titanlegierung, Magnesium, eine Magnesiumlegierung, eine Aluminiumlegierung, Aluminium, AlSiC, aber auch Stahl, rostfreier Stahl oder Edelstahl.

Der Metallstift im Fixiermaterial wird über die gesamte Dicke D des Grundkörpers, der im oben genannten Bereich liegt in die in den Grundkörper eingestanzte Durchgangsöffnung eingelassen, insbesondere eingeglast. Das Einglasen geschieht dadurch, dass zunächst der Metallstift in das Fixiermaterial, beispielsweise einen Glaspfropfen eingeschmolzen wird. Danach wird der Metallstift mit dem Glasmaterial in die Durchgangsöffnung eingelassen und Metallstift, Glasmaterial und Grundkörper erwärmt, derart, dass nach dem Abkühlen das Metall, bevorzugt dass Metall des Grundkörpers auf das Fixiermaterial, beispielsweise den Glaspfropfen aufschrumpft.

Da der Ausdehnungskoeffizient des Grundkörpers größer ist als der des Fixiermaterials, liegt nach dem Abkühlen eine Druckeinglasung vor, insbesondere eine hermetisch dichte Druckeinglasung.

Unter hermetisch dicht wird in dieser Anmeldung verstanden, dass die Helium-Leckrate geringer als 1·10⁻⁸ mbar l/sec ist.

Um eine dauerhafte Druckeinglasung herzustellen, die auch nach Abkühlen und späteren thermischen Zyklen im Betriebszustand dauerhaft dicht ist, wird im Stand der Technik und insbesondere bei Anzündern von Personenschutzeinrichtungen wie Airbags und/oder Gurtstraffern davon ausgegangen, dass die thermischen Ausdehnungskoeffizienten der beteiligten Materialien bestimmte Verhältnisse zueinander ausweisen müssen. Da bei Druckeinglasungen der Grundkörper auf das Glasmaterial, auch Glaskörper genannt, aufschrumpfen soll, muss der Koeffizient der thermische Ausdehnung des Grundkörpers größer sein als derjenige des Glasmaterials. Ebenso soll sich beim Abkühlen der eingeglaste Metallstift nicht aus dem Glasmaterial ablösen, so dass wiederum der Metallstift in bekannten Lösungen einen kleineren Koeffizienten der thermischen Ausdehnung aufweist als das Glasmaterial. Wird in Anzündern von Personenschutzeinrichtungen Edelstahl als Material des Grundkörpers verwendet, wird daher üblicherweise ein Metallstift aus Nickel-Eisen bzw. einer Nickel-Eisen Legierung in das Glasmaterial eingeglast. Aus der EP 0 248 977 A1 ist aber auch ein elektrischer Zündbrückenträger bekannt, bei dem außer einem ringförmigen Grundkörper auch in einem Keramikkörper eingepaßte Kontaktstifte aus Edelstahl bestehen.

Des Weiteren ist die Durchgangsöffnung bei den Durchführungen mit mehr als einem Stift gemäß der US 2007/0187934 A1 außermittig angeordnet.

Das Ausstanzen der Grundkörper aus einem Blechmaterial gemäß der US 2007/0187934 A1 hat Nachteile. Ein Nachteil besteht darin, dass bei einem Stanzen aus einem Bandmaterial, beispielsweise einem Blech des Grundkörpers, ein Anteil an Materialabfall entsteht.

Die DE 10 2010 045 624 A1 schlägt daher vor, den Grundkörper aus einem Drahtmaterial durch ein Kaltumformverfahren herzustellen und den Grundkörper mit einem Freistellungsbereich zu versehen, so dass auch aus dem durch ein Kaltumformverfahren hergestellten Grundkörper die Durchgangsöffnung ausgestanzt werden kann.

Die DE 10 2006 056 077 A1 zeigt eine pyrotechnische Schutzvorrichtung, insbesondere für einen Airbag oder Gurtstraffer, mit einer Durchgangsöffnung in einem Grundkörper, wobei die Durchgangsöffnung in den Grundkörper durch Stanzen eingebracht wird.

Weitere Schriften aus denen Metall-Fixiermaterial-Durchführungen hervorgehen sind beispielsweise die EP 1 491 848 A1, EP 1 455 160 A1, die EP 1 813 906 A1, die EP 2 431 703 A1 oder die DE 10 2006 004036 A1.

Insbesondere bei Metall-Fixiermaterial-Durchführungen mit zwei Metallstiften ist die Durchgangsöffnung in die wenigstens ein Metallstift eingebracht wird, in den meisten Fällen außermittig angeordnet. Außermittige Durchgangsöffnung können Nachteile in einer rationellen Serienfertigung haben.

Aus allen den vorgenannten Anmeldungen betreffend Metall-Fixiermaterial-Durchführungen ist kein Stiftmaterial bekannt geworden, das insbesondere nachdem der Metallstift in das Glasmaterial eingeglast wurde eine sichere Nachverarbeitung, beispielsweise Assemblierung, ermöglicht. Die in der WO 2012/110 245 A1 offenbarten Stiftmaterialien, insbesondere NiFe, neigen bei der rationellen Serienfertigung in automatisierten Produktionsanlagen und/oder bei der Weiterverarbeitung der Metall-Fixiermaterial-Durchführungen zu beispielsweise einem Anzünder und/oder der Montierung des Endprodukts dazu, beispielsweise beim Aufschieben auf einen Stecker, zu verbiegen und im Extremfall sogar durchzubrechen, so dass dadurch beispielsweise unerwünschte Ausschüsse erzeugt werden können .

Aufgabe der Erfindung ist es daher, die Nachteile des Standes der Technik zu vermeiden und eine Metall-Fixiermaterial-Durchführung mit einem Leiter zur Verfügung zu stellen, die sich dadurch auszeichnet, dass sie in der rationellen Serienfertigung mit geringerer Ausschussquote hergestellt werden können und/oder dass sie sicherer assembliert werden können, beispielsweise beim Aufschieben des Endprodukts auf oder in einen Stecker.

Erfindungsgemäß wird diese Aufgabe durch den Gegenstand des unabhängigen Anspruchs 1 gelöst, wobei bei einer Metall-Fixiermaterial-Durchführung mit wenigstens einem Metallstift der wenigstens eineMetallstift zumindest in seinem Kernbereich aus einem Edelstahl nach EN 10020:2000 besteht, wobei der Metallstift sich dadurch auszeichnet, dass der Edelstahl ein ferritischer Edelstahl ist und derart gewählt ist, dass der Metallstift umgerechnet auf die Standarddimensionierung eines Metallstiftdurchmessers von 1,00 ± 0,03 mm und einer Metallstiftlänge von 11,68 ± 0,2 mm, eine maximale elastische Verbiegung von zumindest 0,13 mm, bevorzugt zumindest 0,15 mm, besonders bevorzugt zumindest 0,18 mm, ganz besonders bevorzugt zumindest 0,24 mm

Unter elastische Verbiegung wird im Sinne der Erfindung eine Verbiegung des Metallstifts verstanden, unter welcher bei Wegfall der mechanischen Belastung der Metallstift wenigstens im Wesentlichen wieder in seine ursprüngliche Form zurückkehrt. Es findet dabei mindestens im Wesentlichen keine plastische Verformung statt, oder mit anderen Worten, das Material des Metallstifts befindet sich im genannten Belastungsbereich im Bereich der elastischen Verformung.

Die Metallstiftlänge bezeichnet den Überstand des Metallstiftes von der Unterseite der Einglasung gemessen und ist daher unabhängig von der Länge der Einglasung und/oder der Headerdicke.

Da der Metallstift erfindungsgemäß in eine Durchgangsöffnung eines Grundkörpers in einem glasigen oder glaskeramischen Material eingeschmolzen ist, wird der Metallstift beim Einglasen erhitzt, in der Regel auf Temperaturen von 600°C und mehr, insbesondere 650 °C und mehr. Danach wird er wieder abgekühlt. Dadurch liegt der Edelstahl des Metallstiftes nach dem Einglasen im abgekühlten Zustand nach Erwärmung vor, man spricht dabei allgemein von einem geglühten Zustand oder englisch "annealed". Die Materialeigenschaften des geglühten Edelstahls unterscheiden sich stark von denen des Rohzustands, d.h. des nicht-geglühten Zustands.

Der Metallstift kann als Vollmaterial vorliegen oder als Vollmaterial mit einer Beschichtung. Handelt es sich um einen Metallstift mit einer Beschichtung, so bezeichnet der Kernbereich des Metallstiftes das Vollmaterial, also das Edelstahlmaterial, das von der Beschichtung umgeben ist.

Die Erfindung hat den Vorteil, dass der Edelstahlstift sich durch eine geringe Verbiegbarkeit auszeichnet, insbesondere im geglühten Zustand. Dies bedeutet, dass eine größere mechanische Belastung erforderlich ist, um ihn plastisch zu verformen, als es bei einem bisher verwendeten Nickel-Eisen Stift der Fall ist. Oder mit anderen Worten, bei einer gegebenen mechanischen Belastung unterhalb der plastischen Verformung bleibt der Edelstahlstift elastisch verformbar. Dies kommt unter anderem dem Fertigungsprozess zu Gute, da in Produktionsstraßen die Wahrscheinlichkeit der Verformung des Edelstahlstifts abnimmt. Ebenso kann in der Nachverarbeitung, beispielsweise der Beschichtung oder beim Schleifen der Enden, eine exaktere Positionierung der z.B. der Werkzeuge erfolgen. Ebenso ist es einfacher, einen unverbogenen, d.h. einen nicht von den Sollmaßen abweichenden Metallstift, auf Stecker o.ä. zu schieben.

Besonders bevorzugt ist es, wenn der Edelstahl derart gewählt ist, dass der Metallstift bei einem Standard-Belastungstest im Bereich 3N bis 4N eine Auslenkung von höchstens 0,21 mm zeigt, d. h. die Auslenkung geringer als 0,21 mm ist. Bei einer derart geringen Auslenkung erfolgt lediglich eine elastische Deformation bzw. Verformung, des Metallstiftes, so dass der Metallstift sich wieder in den Ausgangszustand zurückstellt.

Der Standard-Belastungstest dazu ist so gestaltet, dass ein Metallstift mit den Dimensionen 1,00 ± 0,03 mm und einer Metallstiftlänge von 11,68 ± 0,02 mit der genannten mechanischen Belastung senkrecht zur Stiftachse beaufschlagt und die Auslenkung gemessen wird, insbesondere bis zu der Grenze Wmax, bei welcher die Grenze der elastischen Verbiegung erreicht wird. Weist der Metallstift andere Dimensionen auf, ist ein entsprechender Metallstift in der Dimensionierung des Standard-Belastungstest herzustellen oder die Auslenkung entsprechend zu berechnen.

Besonders bevorzugt ist es, wenn der erfindungsgemäße Metallstift so gewählt ist, dass die mechanische Belastung 0,25 % (strain) des zumindest eines Metallstifts einer Spannung von mehr als 450 MPa entspricht, bevorzugt mehr als 480 MPa oder mehr als 500 MPa, insbesondere bevorzugt von 450 MPa bis 700 MPa. Diese Bereiche gelten als besonders vorteilhaft, da eine hohe Spannung zwar eine geringe Verbiegbarkeit, d.h. auch eine höhere Grenze der plastischen Verformung aufweisen, sich aber Materialien mit hoher Spannungen schlechter nachverarbeiten lassen, insbesondere schlechter schleifen lassen. Diese Bereiche sind für geglühte Metallstifte aus Edelstahl höher als für geglühte Metallstifte aus Nickel-Eisen, wie sie aus dem Stand der Technik bekannt sind.

Überraschenderweise wurde bei Verwendung von Edelstahl als Stiftmaterial festgestellt, dass die Auszugskraft des Edelstahl-Metallstiftes aus dem Glasmaterial der Durchgangsöffnung mehr als 250 N beträgt, insbesondere von 250 N bis 400 N, bevorzugt 300 N bis 380 N. Es wird vermutet, dass dies darauf zurückzuführen ist, dass der eingeglaste und damit der geglühte Edelstahl-Metallstift so hart ist, dass er dem Druck der Druckeinglasung besser widersteht. Mit anderen Worten und vereinfacht ausgedrückt, wenn der Grundkörper wie zuvor beschrieben beim Abkühlen auf den Glaskörper in der Durchführungsöffnung aufschrumpft, setzt sich der Druck durch den Glaskörper fort und drückt auf dem Metallstift. Ist dieser weich, kann er diesem Druck nachgeben, so dass die Klemmwirkung in dem Glaskörper schwächer ausfällt als im Falle eines härtere Metallstifts. Die Klemmwirkung ist dabei ein wichtiger Aspekt für die Auszugskraft.

In einer bevorzugten Ausführungsform ist der Edelstahl-Metallstift in einen Grundkörper eingeglast, wobei der Grundkörper ebenfalls aus einem Metall, insbesondere Stahl, rostfreier Stahl, Edelstahl, Titan, einer Titanlegierung, Magnesium, einer Magnesiumlegierung, einer Aluminiumlegierung, Aluminium oder AlSIC bestehen kann.

Besonders vorteilhaft wird als Material des Grundkörpers die gleiche Materialklasse verwendet wie für das Material des Metallstifts, insbesondere des in dem Fixiermaterial angeordneten Metallstift, d.h. Edelstahl-Metallstift und Edelstahl-Grundkörper.

Die Erfinder haben erkannt, dass die Wahl der gleichen Materialklasse eine mögliche elektrochemische Korrosion unterdrücken kann, was vorteilhaft für Produktionsprozesse, insbes. bei der Reinigung und/oder galvanischen Beschichtung sein kann, aber auch zur Langzeitbeständigkeit des Endprodukts, beispielsweise eines Anzünders beitragen kann.

Erfindungsgemäß ist der Edelstahl des zumindest einen Metallstifts (5) ein legierter Edelstahl nach EN 10020:2000, besonders bevorzugt ein chromhaltiger Edelstahl.

Erfindungsgemäß ist der Edelstahl ausgewählt aus der Gruppe der ferritischen Edelstähle.

Dass es mit eingeglasten Metallstiften aus Edelstahl möglich ist, eine dauerhaft stabile Durchführung zu erreichen, ist überraschend, da Edelstähle nicht das zuvor beschriebene Verhältnis der thermischen Ausdehnungskoeffizienten aufweisen. Üblicherweise sind die thermischen Ausdehnungskoeffizienten von Edelstahl nämlich größer als diejenigen des für die Einglasung verwendeten Fixiermaterials, insbesondere des Glas- und/oder Glaskeramikmaterials. Dass dennoch größere Auszugskräfte erreichbar sind als mit den eigentlich in das Verhältnis fallende Nickel-Eisen Stifte ist ein Verdienst der Erfinder und war nicht vorher zu sehen. Es existieren auch hier besonders vorteilhafte Bereiche. So ist es bevorzugt, wenn der Edelstahl so ausgewählt ist, dass der thermische Ausdehnungskoeffizient α _{Metallstift} oder CTE (P) bei einer Temperatur von 650°C im Bereich 9,0 · 10⁻⁶ /K bis 15,0 · 10 ⁻⁶ /K , bevorzugt 11,0 ·10⁻⁶ /K bis 14,0 ·10⁻⁶ /K , bevorzugt 11,5 ·10⁻⁶ /K bis 14,0 ·10⁻⁶ /K oder 11,0 ·10⁻⁶ /K bis 13,5 ·10⁻⁶ /K, besonders bevorzugt 11,5 ·10⁻⁶ /K bis 12,5 ·10⁻⁶ /K liegt.

In einer bevorzugten Ausführungsform hat das glasige oder glaskeramische Fixiermaterial einen thermischen Ausdehnungskoeffizient α_{Glas} bei einer Temperatur bis zu Tg des Fixiermaterials, insbesondere des Glas- und/oder Glaskeramikmaterials, im Bereich 4· 10⁻⁶1/K bis 10,6 ·10⁻⁶1/K. Vorteilhaft ist diese Lage der thermischen Ausdehnung insbesondere in Kombination mit der vorgenannten Lage der thermischen Ausdehnung des Edelstahls.

Weiterhin vorteilhaft ist, wenn der Grundkörper einen thermischen Ausdehnungskoeffizient α_{grundkörper} aufweist, der mindestens 2 ·10⁻⁶ 1/K, bevorzugt 10 ·10⁻⁶ 1/K größer ist als der thermische Ausdehnungskoeffizient α_{Glas} Fixiermaterials, bevorzugt liegt α_{Grundkörper} im Bereich 11 ·10⁻⁶ 1/K bis 18 ·10⁻⁶ 1/K. Gerade in Kombination mit einem oder insbesondere beiden vorgenannten Lagen der thermischen Ausdehnungskoeffizienten des Edelstahls und des Fixiermaterials lässt sich besonders vorteilhaft eine stabile Durchführung, insbesondere Druckeinglasung erreichen.

Der Edelstahl für den Metallstift ist erfindungsgemäß aus der Gruppe der ferritischen Edelstähle ausgewählt. Vorteilhaft ist ferritischer Edelstahl, da dieser besonders rationell zu fertigen ist und/oder besonders rationell Produktionseinrichtungen zuzuführen ist.

In einer bevorzugten Ausführungsform weist der zumindest eine Metallstift zumindest einen Biegepunkt auf. Bevorzugt ist der Metallstift so gebogen, dass ein axialer Versatz S des Bereichs des Metallstifts in der Durchgangsöffnung und des Anschlussbereichs an dessen gegenüberliegenden Ende vorliegt. Dadurch kann insbesondere günstig eine mittig im Grundkörper angeordnete Durchgangsöffnung realisiert werden.

Darüber hinaus hat ein gebogener Metallstift den Vorteil, insbesondere ein S-förmig gebogener Metallstift, dass er bei den erfindungsgemäßen Edelstählen als Material für den Metallstift bei der Montage auf Stecker eine Art Federfunktion bereitstellen kann, welche beim Aufschieben auf den Stecker die Wahrscheinlichkeit der Beschädigung des Steckersystems reduziert, beispielsweise das Herausdrücken von Metallhülsen aus Kunststoffhalterungen. Darüber hinaus werden mechanische Belastungsspitzen von dem Glasmaterial der Durchführung abgehalten.

Der gebogene Metallstift ist mit den erfindungsgemäßen Edelstählen schwieriger herzustellen als mit den bisher verwendeten NiFe Stählen, da die Verbiegung nach dem Glühen stattfindet und der geglühte Edelstahl, wie anhand der beschriebenen Festigkeitswerte ersichtlich ist, nur mit größerem Kraftaufwand plastisch verformt werden kann.

Bevorzugt weist die Metall-Fixiermaterial-Durchführung zumindest einen weiteren Metallstift auf, der elektrisch leitfähig mit dem Grundkörper verbunden ist, insbesondere mittels einer Lotverbindung oder einer Schweißverbindung. Dadurch wird ein direkter elektrischer Kontakt des Grundkörpers mit dem zweiten Metallstift hergestellt, so dass auf eine zweite Durchführungsöffnung im Grundkörper verzichtet werden kann.

In einer bevorzugten Ausführungsform besteht der elektrisch leitfähig mit dem Grundkörper verbundene Metallstift zumindest in seinem Kernbereich aus einem Nicht-Edelstahl, insbesondere aus NiFe, und ist mittels einer Schweißverbindung mit dem Grundkörper verbunden. Vorteilhaft liegt dabei dieser Metallstift im nicht-geglühten Zustand vor, zumindest abseits des Bereichs der Schweißverbindung, die beim Verschweißen erhitzt wurde. Diese Materialwahl hat den Vorteil, dass der Nicht-Edelstahl im Rohzustand mechanisch wie beschrieben belastbarer ist als der geglühte Edelstahl. So weist diese Ausführungsform die besten mechanischen Festigkeitswerte auf. Allerdings ist es aufwendiger, die Schweißverbindung herzustellen, als den zweiten Metallstift an den Grundkörper anzulöten.

In einer vorteilhaften Ausführungsform weist der weitere Metallstift ebenfalls zumindest einen Biegepunkt auf. Bevorzugt ist der weitere Metallstift so gebogen, dass ein axialer Versatz des mit dem Grundkörper verbundenen Bereichs des Metallstifts und des Anschlussbereichs in an dessen gegenüberliegenden Ende vorliegt.

Um ein Verlöten des Metallstiftes aus Edelstahl mit metallischen Loten zu begünstigen, weist der zumindest eine in die Durchgangsöffnung eingeglaste Metallstift und/oder der mit dem Grundkörper elektrisch leitfähig verbundene Metallstift vorteilhaft eine Nickelbeschichtung auf. Bevorzugt liegt die Nickelbeschichtung zumindest im Bereich der Einglasung und/oder im Bereich der Kopffläche des Metallstifts in der Einglasung und/oder im Bereich der elektrisch leitfähigen Verbindung mit den Grundkörper vor. Zusätzlich oder alternativ zur Nickelschicht kann noch eine Goldschicht vorgesehen sein.

Insbesondere vorteilhaft befindet sich die Goldschicht zumindest bereichsweise auf der Nickelschicht.

Somit sind insbesondere die Nickel-Beschichtungen möglich:
- am eingeglasten Metallstift in einem Bereich, der mit einer Goldschicht auf zumindest Bereichen der Nickelschicht versehen ist, insbesondere in dem Anschlussbereich am Ende des Metallstifts,
   und/oder
- am eingeglasten Metallstift in einem Bereich, der mit dem Fixiermaterial in Kontakt steht,
   und/oder
- am elektrisch leitfähig mit dem Grundkörper verbundenen Metallstift in einem Bereich, der mit einer Goldschicht auf zumindest Bereichen der Nickelschicht versehen ist, insbesondere in dem Anschlussbereich am Ende des Metallstifts,
   und/oder
- am elektrisch leitfähig mit dem Grundkörper verbundenen Metallstift in einem Bereich, an dem der Metallstift mittels einem metallischen Lotmaterial mit dem Grundkörper verbunden ist.

Um einen sicheren elektrischen Anschluss zu gewährleisten ist der zumindest eine in der Durchgangsöffnung eingeglaste Metallstift und/oder der mit dem Grundkörper elektrisch leitfähig verbundene Metallstift bevorzugt zumindest bereichsweise mit Gold beschichtet. Bevorzugt liegt die Goldschicht zumindest im Anschlussbereich und/oder des elektrisch leitfähig mit dem Grundkörper verbundenen Metallstifts vor. Der Anschlussbereich ist insbesondere der Bereich, in welche der Metallstift in beispielsweise ein Steckersystem eingeschoben wird und/oder wo dieser mit Kontakten eines Steckersystems kontaktiert wird.

Um eine möglichst einfach zu verarbeitende bzw. zu assemblierende Metall Fixiermaterial-Durchführung mit einem Metallstift zur Verfügung zu stellen, ist bei einer derartigen Metall-Fixiermaterial-Durchführung der Metallstift derart ausgelegt, dass er im geglühten Zustand in einem Testsystem mit der Metallstiftlänge L von 11,68 mm beim senkrechten Beaufschlagen am Endpunkt L bei einer Kraft Fₘₐₓ bricht, wobei Fₘₐₓ mehr als 2,2 N beträgt. Der Metallstift kann auch so ausgelegt sein, dass er im geglühten Zustand in einem Testsystem mit der Metallstiftlänge L von 11,68 mm beim senkrechten Beaufschlagen am Endpunkt L bis zu einer Maximalauslenkung Wₘₐₓ elastisch verformbar ist, wobei Wₘₐₓ mehr als 0,15 mm beträgt, insbesondere von 0,15 mm bis 0,4 mm. Wₘₐₓ bezeichnet somit die Grenze der elastischen Verformung. Bei einer Belastung über Wₘₐₓ hinaus erfolgt eine plastische Verformung, also eine dauerhafte Verbiegung des Metallstifts.

Weist ein realer Metallstift andere Dimensionierungen auf als die als Testsystem genannten, wird dieser zum Vergleich in den Dimensionen des Testsystems hergestellt und/oder dessen Messergebnisse werden so umgerechnet, dass sie der Dimensionierung des Testsystems entsprechen.

Der erfindungsgemäße Metallstift aus Edelstahl weist nach dem Einglasen und einer Erwärmung auf 600° C bzw. 650°C eine wesentlich höhere Steifheit als ein NiFe-Stift auf. Dies ist darauf zurückzuführen, dass NiFe durch die hohe Temperatur erweicht, Edelstahl dagegen nicht. So ist bei Edelstahl eine um 50% höhere Belastung des Stiftes nach dem Einschmelzen möglich als beispielsweise bei NiFe.

Metall-Fixiermaterial-Durchführungen mit Metallstiften aus Edelstahl zeichnen sich durch eine sehr hohe mechanische Stabilität des Metallstiftes aus. Die hohe mechanische Stabilität verhindert ein Verbiegen, insbesondere dauerhaftes oder plastisches Verbiegen des Metallstiftes bei der Montage und der Nachverarbeitung. Die Verwendung von Edelstahl als Stiftmaterial stellt sicher, dass die mechanische Stabilität gegenüber Stiften beispielsweise aus einem NiFe-Material stark erhöht ist.

Des Weiteren zeichnet sich der erfindungsgemäße Metallstift in einer Metall-Fixiermaterial-Durchführung für Anzünder von Airbags und Gurtspannern dadurch aus, dass er unerwarteter Weise eine sehr hohe Auszugskraft von mehr als 250 N, insbesondere 250 N bis 400 N, bevorzugt 300 N bis 380 N aufweist. Dies war für den Fachmann überraschend, da der thermische Ausdehnungskoeffizient der Stifte aus einem Edelstahl im Bereich von 11,0 ■ 10 ⁻⁶ /K bis 13,5·10 ⁻⁶ /K bei 650°C liegt und somit in einem anderen Bereich wie das Glasmaterial und das umgebende Metall der Metall-Fixiermaterial-Durchführung, so dass geringere Auszugskräfte erwartet wurden, welche die Verwendung von Metallstiften aus Edelstahl als nachteilig erwarten ließ. Es wird vermutet, dass diese höhere Auszugskraft zurückzuführen ist auf eine bessere, chemische Adhäsion des Stiftes in dem Glasmaterial aufgrund des gewählten Stiftmaterials.

In einer weitergebildeten Ausführungsform ist der Edelstahl des wenigstens einen Metallstifts ausgewählt aus der Gruppe Edelstähle, deren Übergangspunkt zwischen elastischer und plastischer Verformung im geglühten Zustand weniger als 50 % unter dem Übergangspunkt zwischen elastischer und plastischer Verformung im Rohzustand liegt.

Durch eine derartige Auswahl wird sichergestellt, dass der Metallstift nach dem Einbringen in das Fixiermaterial, insbesondere in das Glasmaterials und Einsetzen in die Öffnung des Grundkörpers der Metall-Fixiermaterial-Durchführung beim anschließenden Erwärmen des Grundkörpers auf mindestens 600°C bzw. 650°C, nicht derart erweicht, dass der Metallstift bei mitunter auftretenden mechanischen Belastungen plastisch verformbar wird. Eine zu starke Reduzierung des Übergangspunkts von elastischer zu plastischer Verformung des geglühten Metallstifts würde dazu führen dass die Steifigkeit des Metallstiftes und damit die mechanische Stabilität erheblich reduziert wären.

Während der Übergangspunkt vom elastischen auf das plastische Verhalten bei einem ferritischen Edelstahl im Rohzustand bei einer Spannung von 600 MPa liegt und auf 500 MPa durch Erwärmen auf 600°C im Spannungs- Dehnungsdiagramm sinkt, fällt bei NiFe der Übergangspunkt von 700 MPa durch Erwärmen auf 600°C auf 300 MPa im Spannungs-Dehnungsdiagramm ab. Dies bedeutet, dass die NiFe Stähle im ungeglühten Zustand durchaus mechanisch stabiler sind als die erfindungsgemäßen Edelstähle. Im geglühten Zustand sind jedoch die Edelstähle mechanisch belastbarer, insbesondere bis hin zu höheren Belastungen elastisch verformbar.

Da Edelstahl im geglühten Zustand weniger stark erweicht als NiFe, ist die mechanische Weiterverarbeitung des Edelstahls im Vergleich zu NiFe erschwert. Dies manifestiert sich in einem erschwerten Biegeverhalten, insbesondere wenn z.B. die S-förmige Biegung der Metallstifte erzeugt werden soll, sowie einer schwierigeren Bearbeitung in Bezug auf die Formung des Endes des Metallstifts oder der Metallstifte, beispielsweise des Schleifens oder Prägens eines Radius. Vor allem die unpassende Lage der thermischen Ausdehnungskoeffizienten und die erschwerte Nachbearbeitbarkeit haben den Fachmann davon abgehalten, Edelstahl als Material für den Metallstift anstelle von NiFe einzusetzen.

Ein Vorteil von Edelstahl als Stiftmaterial gegenüber herkömmlichen Materialien wie NiFe ist, dass in der Kombination mit einem Grundkörper aus Edelstahl bei angeschlossenem Brückendraht oder bei der Belegung mit elektrisch leitfähigen Filmen praktisch keine galvanische Korrosion auftritt. Dies ist darauf zurückzuführen, dass die Differenz der elektrochemischen Potentiale zwischen Edelstahl-Grundkörper und Edelstahl-Metallstift gering ist. Angestrebt wird ein Absolutbetrag der Differenz der elektrochemischen Potentiale von Metallstift, insbesondere dem eingeglasten Metallstift, und Grundkörper von bis zu höchstens 0,3 V. Das heißt, der Absolutbetrag der Differenz der elektrochemischen Potentiale von Metallstift, insbesondere dem eingeglasten Metallstift, und Grundkörper liegt vorteilhaft im Bereich von 0 bis 0,3 V. Dadurch kann erreicht werden, dass praktisch keine galvanische Korrosion auftritt. Wird hingegen ein NiFe-Stift eingesetzt, so wandern Elektronen vom NiFe-Stift zum Material des Grundkörpers, beispielsweise aus austenitischem Edelstahl und galvanische Korrosion tritt auf. Bei Verwendung von beispielsweise ferritischem Edelstahl als Material für den Metallstift ist das elektrischen Potential von Metallstift und dem aus austenitischen Edelstahl bestehenden Grundkörper praktisch gleich groß und galvanische Korrosion tritt im Gegensatz zu einem NiFe-Stift nicht auf.

Ebenso kann durch die Erfindung angegeben werden, dass die Auswahl der Materialien für Grundkörper und/oder Metallstift vorteilhaft anhand der elektrochemischen Potentiale gegenüber Meerwasser erfolgen kann. Dieses Potential gegen Meerwasser ist ein gutes Maß für die Beurteilung der Beständigkeit gegenüber elektrochemischen Korrosionsangriffen, weil im Betriebszustand, insbesondere über lange Lagerungs- oder Betriebszeiten, die sich auf der Oberfläche der Durchführung bildende Filme ähnlich korrosiv wie Meerwasser sein können.

Dieser erfinderischen Konzept folgend sind Materialien für die Auswahl des Grundkörper und/oder des zumindest einen Metallstifts vorteilhaft, insbesondere des in dem Fixiermaterial befindlichen Metallstifts, insbesondere Edelstähle, deren Absolutbetrag des elektrochemischen Potentials gegenüber Meerwasser höchstens 0,36 V beträgt, d.h. entsprechend in einem Bereich von 0 bis 0,36 V liegen.

Dadurch, dass Edelstahl als Stiftmaterial zu Edelstahl als Material des Grundkörpers eine höchstens geringe elektrochemische Potentialdifferenz aufweisen, kann man eine Metall-Fixiermaterial-Durchführung für Anzünder von Airbags und/oder Gurtstraffern mit zumindest einem Metallstift, der in eine Durchgangsöffnung eines Grundkörpers in einem glasigen oder glaskeramischen Fixiermaterial eingeschmolzen ist, zur Verfügung stellen, bei der wenigstens der Metallstift und der Grundkörper aus einer kompatiblen Materialkombination bestehen, so dass bei installierter Zündbrücke eine Anoden- und/oder Kathodenreaktion auf der Oberseite des Grundkörpers unterdrückt wird, wobei die Oberseite der Durchführung definiert ist als die Seite einer anzubringenden Zündbrücke und die Unterseite definiert ist als die Seite der elektrischen Anschlüsse, d. h. die Seite der Metall-Fixiermaterial-Durchführung, aus der die Metallstifte herausragen.

Besonders bevorzugt ist es, wenn zumindest ein Metallstift und der Grundkörper im Wesentlichen das gleiche elektrochemische Potential aufweisen, so dass über einen auf der Wasseroberfläche absorbierten Wasserfilm bei installierter Zündbrücke kein Elektronenfluss über die Zündbrücke erfolgt. Bevorzugt beträgt der Absolutbetrag des Unterschieds im elektrochemischen Potential von Grundkörper und Edelstahlstift zwischen 0,3 V bis 0,0 V, d. h. der Grundkörper hat beispielsweise ein Potential von 0,07 V, der Metallstift von 0,02 V, so dass der Unterschied 0,05 V beträgt und somit so gut wie kein Elektronenfluss über die Zündbrücke vom Metallstift zum Grundkörper und/oder über leitende Filme erfolgt.

Besonders bevorzugt ist es, wenn der Cr-Anteil im Edelstahl im Bereich von 10 Gewichtsprozent bis 30 Gewichtsprozent, bevorzugt 15 Gewichtsprozent bis 25 Gewichtsprozent, liegt. So wird beispielsweise bei einem Anteil von 20 Gewichtsprozent Chrom ein sehr niedriger linearer Ausdehnungskoeffizient von ungefähr 10 ·10 ⁻⁶ /K bei 0 bis 40°C erreicht. Der niedrige Ausdehnungskoeffizient bei 40°C korreliert auch mit einem niedrigen Ausdehnungskoeffizienten bei der Einglasungstemperatur, üblicherweise 600°C bzw. 650°C.

Um ein Verlöten des Metallstiftes aus Edelstahl zu ermöglichen, ist vorgesehen, dass der Metallstift wie beschrieben zumindest bereichsweise mit einer Ni-Schicht und/oder einer Goldschicht versehen wird. Hierbei kann die Nickelschicht auch unterhalb der Goldschicht vorgesehen sein. Auch eine direkte Vergoldung des Edelstahls ohne eine Nickel-Zwischenschicht ist möglich.

Überraschenderweise wurde festgestellt, dass bei Verwendung von Edelstahl als Material für den Metallstift eine Auszugskraft des Metallstiftes im Bereich von 250 N bis 400 N, bevorzugt 300 N bis 380 N aus dem Glasmaterial zur Verfügung gestellt wird. Diese Auszugskraft ist unerwarteter Weise etwa 50 % größer als beispielsweise bei einem NiFe-Metallstift.

Einen besonders stabilen Metallstift umfasst die Metall-Fixiermaterial-Durchführung, wenn der Metallstift derart ausgelegt ist, dass er im geglühten Zustand in einem Testsystem mit der Metallstiftlänge L von 11,68 mm bei senkrechtem Beaufschlagen am Endpunkt L bei einer Kraft Fₘₐₓ bricht, wobei Fₘₐₓ mehr als 2,2 N beträgt und/oder wobei der Metallstift so ausgelegt ist, dass er im post-erhitzten Zustand in einem Testsystem mit der Metallstiftlänge L von 11,68 mm bei senkrechtem Beaufschlagen am Endpunkt L bei einer Maximalauslenkung Wₘₐₓ bricht, wobei der Wₘₐₓ mehr als 0,15 mm beträgt, insbesondere von 0,15 mm bis 0,4 mm. Ein derart stabiler Metallstift wird insbesondere dann ausgebildet, wenn als Material für den Metallstift Edelstahl, insbesondere chromhaltiger Edelstahl, eingesetzt wird.

Bislang wurde der Fachmann vom Einsatz von Edelstählen in Druckglasdurchführungen, beispielsweise zur Verwendung in Airbag-Zündern abgehalten, da der thermische Ausdehnungskoeffizient dieser Stähle mit 11,0 bis 13,5 10⁻⁶/K bei 650°C deutlich höher ist als der Ausdehnungskoeffizient des Glases, der im Bereich 10,6 bis 6,1 10⁻⁶ /K liegt. Die Erfinder haben herausgefunden, dass überraschenderweise trotz des größeren Ausdehnungskoeffizienten des Leiters, der oberhalb des Glases liegt, entgegen dem Stand der Technik, der vorschreibt, dass bei Glas-Metall-Durchführungen die thermische Ausdehnung des Leiters nicht höher sein darf als die des verwendeten Glases, um eine ausreichende Dichtheit zur Verfügung zu stellen, auch im Falle der Ausdehnungskoeffizienten des Metallstiftes größer als der des Glases ist, eine dichte Einglasung dann zur Verfügung gestellt wird, wenn ein positiver Fugendruck vom Grundkörper auf das Glas ausgeübt wird. Besonders bevorzugt ist es, wenn der Fugendruck größer 30 MPa, bevorzugt größer 50 MPa, insbesondere größer 100 MPa, ist. Bei derartigen Fugendrücken wird eine zuverlässige Einglasung erreicht. Der Fugendruck ist der Druck an der Stelle des der Übergangs des Grundkörpers zum Glasmaterial. Er wirkt bei einer Druckeinglasung üblicherweise senkrecht von der Innenwand der Durchgangsöffnung auf den Glaskörper. Er ist ein wesentlicher Faktor für die Auszugskräfte des Glaskörpers aus dem Grundkörper.

Ein hoher Fugendruck wird dann zur Verfügung gestellt, wenn vom Grundkörper als Außenleiter genügend Druckvorspannung auf das Glas aufgeprägt wird. Der sich dann ergebende Fugendruck zwischen Glas und Innenleiter stellt sich beim Abkühlen der Durchführung nach dem Einschmelzen ein. Ist dieser Fugendruck deutlich positiv, d.h. größer 30 MPa bzw. größer 50 MPa, insbesondere größer 100 MPa, so bleibt der Übergang zwischen Glas und Metall, d. h. der Übergang von Glas zum Metallstift geschlossen und damit dicht, obwohl der Ausdehnungskoeffizient des Metallstiftes größer als der des Glases ist.

Der Fugendruck hängt direkt vom Dehnungsunterschied von Glas und dem umgebenden Metall ab. Des Weiteren sind auch noch Abhängigkeiten von der Geometrie denkbar. Insbesondere vorteilhaft muss die Fläche des Grundkörpers außerhalb der Durchgangsöffnung größer sein als die Fläche der Durchgangsöffnung selbst. Der Fugendruck ist eine Flächenpressung. Der Fugendruck drückt aus, mit welcher Kraft pro Flächeneinheit ein erster Körper auf einen zweiten drückt.

Um den notwendigen Fugendruck aufzubringen, beträgt der Unterschied zwischen dem Ausdehnungskoeffizienten des Grundkörpers und dem Ausdehnungskoeffizienten des Glases mindestens 2 ppm/K, bevorzugt mindestens 4 ppm/K, wobei der Ausdehnungskoeffizient a_{Grundkörper} höher ist als der Ausdehnungskoeffizient des Glases α_{Glas}. In einer besonders bevorzugten Ausführungsform wird der Ausdehnungskoeffizient des Metallstiftes α_{Metallstift} so gewählt, dass der Ausdehnungskoeffizient des Metallstiftes 1,1 mal größer als der Ausdehnungskoeffizient α_{Glas} des Glases ist. In einer besonders bevorzugten Ausführungsform liegt der Ausdehnungskoeffizient im Bereich 1.1 · α_{Glas} bis 2 · α_{Glas}.

Um den notwendigen Druck des Grundkörpers auf das Glasmaterial aufzubringen, und Dichtheit zu garantieren, ist vorgesehen, dass der Grundkörper aus einem nickelfreien, rostfreien, chemisch beständigen Stahl (Edelstahl) besteht.

Vorteilhaft ist es, wenn es sich bei dem Grundkörper, der auch ein Außenleiter sein kann, um einen austenitischen Edelstahl handelt, der sich durch eine gute Verschweißbarkeit auszeichnet.

Neben einem geraden Metallstift kann auch vorgesehen sein, dass der Metallstift der Metall-Fixiermaterial-Durchführung nicht gerade ausgebildet ist, sondern gebogen ist.

Das Fixiermaterial der Metall-Fixiermaterial-Durchführung ist ein Glas- oder Glaskeramikmaterial. Der Ausdehnungskoeffizient der eingesetzten Glasmaterialien α_{Glas} liegt im Bereich 4· 10 ⁻⁶ /K bis 10,6 ·10 ⁻⁶ /K, bevorzugt 6,1 ■ 10⁻⁶ /K bis 10,6 10⁻⁶ /K.

Der Grundkörper der Metall-Fixiermaterial-Durchführung in die der Metallstift eingeglast wird, umfasst eine Öffnung wie beispielsweise in der EP 1 813 906 A1, der EP 1 455 160 A1 oder der EP 2 431 703 A1 beschrieben, die auf unterschiedliche Art und Weise erhalten werden kann. Eine Möglichkeit ist ein Kaltumformverfahren wie in der EP 2 431 703 A1 niedergelegt, wobei die Öffnung in den Grundkörper durch Stanzen eingebracht wird.

Bei Druckglasdurchführungen, wie sie bei Anzündern von Airbags und Gurtspannern zum Einsatz kommen, ist das Material des Grundkörpers so gewählt, dass der Ausdehnungskoeffizient α_{Grundkörper} größer ist als der des Glasmaterials, so dass ein Druck auf das Glasmaterial ausgeübt wird, ergebend eine Druckeinglasung. Vorteilhaft ist bei einer Druckeinglasung ein Grundkörper aus austenitischem Edelstahl mit einem Ausdehnungskoeffizient von α = 18,3·10⁻⁶ /K.

Ein weiterer über die eigentliche Erfindung hinausgehender Aspekt ist es, eine Metall-Fixiermaterial-Durchführung, insbesondere für Anzünder von Airbags und/oder Gurtstraffern zur Verfügung zu stellen, bei der galvanische Korrosion nur in geringem Umfang auftritt. Dieser Aspekt wird dadurch gelöst, dass der wenigstens eine Metallstift und der Grundkörper der Durchführung aus einer kompatiblen Materialkombination bestehen, derart, dass bei installierter Zündbrücke oder bei Belegung der Oberseite mit einem leitfähigen Film eine Anoden- und/oder Kathodenreaktion auf der Oberseite des Grundkörpers nicht oder nur in geringem Umfang auftritt.

Besonders bevorzugt ist es, wenn der zumindest eine Metallstift und der Grundkörper ein elektrochemisches Potential aufweisen und der Absolutbetrag des Unterschieds der elektrochemischen Potentiale von Metallstift und Grundkörper höchstens 0,3 V beträgt. Bevorzugt sind die elektrochemischen Potential von Metallstift und Grundkörper im Wesentlichen gleich. Insbesondere liegt der Absolutbetrag des Unterschieds des elektrochemischen Potentials des Metallstiftes und des Grundkörpers im Bereich 0,1 V bis 0,0 V, bevorzugt von 0,05 V bis 0,0 V. Der Absolutbetrag des Unterschieds des elektrochemischen Potentials des Metallstiftes und/oder des Grundkörpers gegenüber Meerwasser beträgt bevorzugt höchstens 0,36 V und liegt insbesondere im Bereich 0,36 V bis 0,0 V.

Der zumindest eine Metallstift (5) besteht insbesondere zumindest in seinem Kernbereich sowie der Grundkörper zumindest an seiner Oberseite aus einem Edelstahl nach EN 10020:2000.

In einer weitergebildeten Ausführungsform sind der Edelstahl des Metallstiftes und des Grundkörpers so gewählt, dass der Edelstahl des Metallstifts und der Grundkörper einen Passivierungsfilm auf ihrer Oberfläche ausbilden, bevorzugt gegenüber einem absorbierten Wasserfilms.

Insbesondere besteht der wenigstens eine Metallstift zumindest in seinem Kernbereich aus einem Edelstahl nach EN 10020:2000, dessen thermischer Ausdehnungskoeffizient α_{Metallstift} bei einer Temperatur von 650°C im Bereich von 9 bis 15, bevorzugt von 11,0 · 10⁻⁶ /K bis 14,0·10⁻⁶ /K, bevorzugt 11,5 · 10⁻⁶ /K bis 14,0·10⁻⁶ 1/K oder 11 · 10⁻⁶ /K bis 13,5 ·10⁻⁶ 1/K, besonders bevorzugt 11,5 · 10⁻⁶ /K bis 12,5·10⁻⁶ 1/K liegt.

Das glasige oder glaskeramische Fixiermaterial weist bevorzugt einen thermischen Ausdehnungskoeffizient α_{Glas} bei einer Temperatur bis zu Tg des Fixiermaterials im Bereich 4· 10⁻⁶ 1/K bis 10,6 -10⁻⁶ 1/K auf.

Besonders bevorzugt ist es, wenn der Grundkörper einen thermischen Ausdehnungskoeffizient α_{Grundkörper} aufweist, der mindestens 2 ·10⁻⁶ 1/K, bevorzugt 10 ·10⁻⁶ 1/K höher ist als der thermische Ausdehnungskoeffizient α_{Glas} des Glases, bevorzugt im Bereich 11 ·10⁻⁶ 1/K bis 18 ·10⁻⁶ 1/K.

Bei dem Edelstahl des zumindest einen Metallstifts (5) handelt es sich erfindungsgemäß um einen legierten Edelstahl nach EN 10020:2000, besonders bevorzugt einen chromhaltiger Edelstahl. Erfindungsgemäß ist der Edelstahl ausgewählt aus der Gruppe der ferritischen Edelstähle. Die Auszugskraft des Metallstiftes aus dem Glasmaterial der Durchgangsöffnung (11) beträgt vorteilhafterweise mehr als 250 N, insbesondere von 250 N bis 400 N, bevorzugt 300 N bis 380 N.

Insbesondere besteht der Grundkörper aus einem Metall, insbesondere Stahl, rostfreier Stahl, Edelstahl, Titan, einer Titanlegierung, Magnesium, einer Magnesiumlegierung, eine Aluminiumlegierung, Aluminium oder diese im Wesentlichen umfasst. Ganz besonders bevorzugt besteht der Grundkörper zumindest im Wesentlichen aus Edelstahl der Typen 316, 317, 302, 304, 321, 317, 430, 410 und/oder 416.

Der Metallstift umfasst insbesondere umgerechnet auf die Standarddimensionierung eines Metallstiftdurchmessers von 1,00 ± 0,03 mm und einer Metallstiftlänge von 11,68 ± 0,2 mm eine maximale elastische Verbiegung Wₘₐₓ von zumindest 0,13 mm , bevorzugt zumindest 0,15 mm, besonders bevorzugt zumindest 0,18 mm, ganz besonders bevorzugt zumindest 0,24 mm.

Der elektrisch leitfähig mit dem Grundkörper verbundene Metallstift besteht insbesondere zumindest in seinem Kernbereich aus einem Nicht-Edelstahl, insbesondere aus NiFe, und wobei dieser Metallstift mittels einer Schweißverbindung mit dem Grundkörper verbunden ist.

In einer Fortbildung der Erfindung kann vorgesehen sein, dass der zumindest eine in der Durchgangsöffnung eingeglaste Metallstift (5) und/oder der mit dem Grundkörper elektrisch leitfähige Metallstift (6) mit Nickel beschichtet ist. Bevorzugt liegt die Nickelschicht in Bereichen der Metallstifte vor, ausgewählt aus der Gruppe einschließlich Kombinationen derselben:
- am eingeglasten Metallstift in einem Bereich, der mit einer Goldschicht auf zumindest Bereichen der Nickelschicht versehen ist, insbesondere in dem Anschlussbereich am Ende des Metallstifts ,
- am eingeglasten Metallstift in einem Bereich, der mit dem Fixiermaterial in Kontakt steht,
- am elektrisch leitfähig mit dem Grundkörper verbundenen Metallstift in einem Bereich, der mit einer Goldschicht auf zumindest Bereichen der Nickelschicht versehen ist, insbesondere in dem Anschlussbereich am Ende des Metallstifts ,
- am elektrisch leitfähig mit dem Grundkörper verbundenen Metallstift in einem Bereich, an dem der Metallstift mittels einem metallischen Lotmaterial mit dem Grundkörper verbunden ist.

Alternativ oder zusätzlich kann vorgesehen sein, dass der zumindest eine in der Durchgangsöffnung eingeglaste Metallstift und/oder der mit dem Grundkörper elektrisch leitfähige Metallstift mit Gold beschichtet ist. Bevorzugt liegt die Goldschicht zumindest im Anschlussbereich des Metallstifts und/oder des elektrisch leitfähig mit dem Grundkörper verbundenen Metallstifts vor, welcher dem im und/oder am Grundkörper befindlichen Ende des jeweiligen Metallstifts gegenüber liegt.

Die Erfindung soll nachfolgend anhand der Figuren sowie der Ausführungsbeispiele, ohne Beschränkung hierauf detailliert beschrieben werden.

Es zeigen:
Fig. 1a: eine Durchführung gemäß der Erfindung eingesetzt in einen Airbag-Zünder
Fig. 1b: eine Metall-Fixiermaterial-Durchführung bspw. gemäß der EP 2 270 417 A1 mit einem erfindungsgemäßen Metallstift
Fig. 2: Versuchsaufbau zur Ermittlung der Biegesteifigkeit
Fig. 3a: Ausdehnungskoeffizienten von Glas, Grundkörper und Metallstift in einer Metall-Fixiermaterial-Durchführung gemäß dem Stand der Technik
Fig. 3b: Ausdehnungskoeffizienten von Glas, Grundkörper und Metallstift in einer Metall-Fixiermaterial-Durchführung gemäß der Erfindung
Fig. 4: Spannungs-/Dehnungskurve für NiFe und Edelstahl (AISI 430) erhitzt und nicht erhitzt
Fig. 5: Gehäusebauteil in der Draufsicht mit Öffnung und in der Öffnung eingeglastem Metallstift
Fig. 6: Auszugskräfte für Metallstifte aus NiFe/Edelstahl
Fig. 7: Abhängigkeit der Ausdehnungskoeffizienten der Edelstähle vom Cr-Anteil
Fig. 8: Chromgehalt bei Edelstählen
Fig. 9: Chrom- und Nickeläquivalente bei Edelstählen
Fig. 10a: Kopf einer Metall-Fixiermaterial-Durchführung mit Brückendraht
Fig. 10b und 10c: chemische Reaktion aufgrund unterschiedlicher elektrochemischer Potentiale im Stand der Technik und gemäß der Erfindung
Fig. 11: Elektrochemische Potentiale einer Auswahl von Materialien

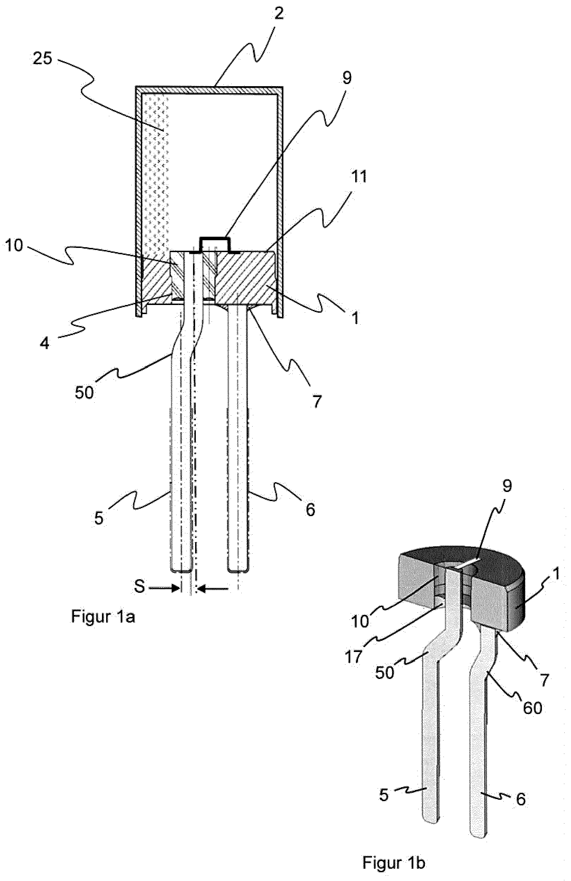

Figur 1a verdeutlicht anhand eines Axialschnittes eine beispielhafte Ausführung einer Metall-Fixiermaterial Durchführung 1, vorzugsweise für den Einsatz in einem Anzünder bzw. Zündvorrichtung eines Airbags oder anderen Personenschutzeinrichtungen wie Gurtstraffern, wie beispielsweise in der EP 2 270 417 A1 beschrieben. Die Metall-Fixiermaterial-Durchführung umfasst ohne Beschränkung hierauf zwei Metallstifte 5, 6, die als erfindungsgemäße Metallstifte ausgebildet sind und als Material des Metallstiftes einen Edelstahl umfassen. Die Metall-Fixiermaterial-Durchführung weist einen Grundkörper 1 auf, mit welchem in dieser Ausführungsform einer der zwei zueinander parallele Metallstifte 5 und 6 elektrisch verbunden ist. Die beiden Metallstifte 5 und 6 sind in der dargestellten Ausführungsform parallel zueinander angeordnet. Dabei fungiert einer als Leiter, während der zweite auf Masse gelegt wird. Im dargestellten Fall fungiert der erste Metallstift 5 als Leiter und der Metallstift 6 als Massestift. Der Massestift 6 ist mit dem Grundkörper 1 beispielsweise durch eine Lotverbindung 7 mittels eines Lotmaterials elektrisch leitend verbinden. Die Breite der beiden Metallstifte liegt üblicherweise im Bereich 0,98 bis 1,05 mm, vorteilhaft bei 1,0 mm.

Wenigstens einer der Metallstifte, insbesondere der als Leiter fungierende Metallstift 5 wird durch den Grundkörper 1 geführt. Der Metallstift 5 ist dazu auf einem Teil seiner Länge in ein Fixiermaterial 10, insbesondere einem aus einer Glasschmelze erkalteten Glaspfropfen eingeschmolzen. Der Metallstift 5 ragt wenigstens auf einer Seite über die Stirnseite des Glaspfropfens 10 hinaus, üblicherweise auf der Unterseite des Grundkörpers, und schließt in der dargestellten Ausführungsform nach Beendigung der Fertigung mit der zweiten Stirnseite , die mit der Oberfläche 11 des Grundkörpers 1 in einer Ebene liegt, des Glaspfropfens 6 bündig ab. Dazu kann der Metallstift 5 während des Einschmelzens derart in der Durchgangsöffnung 4 angeordnet werden, dass dieser zunächst über den Grundkörper 1 hinausragt. Nach dem Einschmelzen bzw. Vergießen kann ein Abschleifen des Metallstiftes 5 und ggfls. des überstehenden erkalteten Fixiermaterials 10 erfolgen, so dass dieser und/oder diese bündig zusammen mit der Stirnseite des Glaspfropfens 10 und der Oberfläche 11 des Grundkörpers 1 sind. Auch andere Varianten sind denkbar. Das Einschmelzen des Metallstiftes in das Glasmaterial und des Glasmaterials in den Grundkörper erfolgt üblicherweise bei Temperaturen von 600°C oder 650 °C und mehr, abhängig von dem verwendeten Glasmaterial. Aufgrund des höheren thermischen Ausdehnungskoeffizienten des Materials des Grundkörpers von α_{Grundkörper} ~ 18,3 · 10⁻⁶ /Kgegenüber dem Glasmaterial mit α_{Glasmaterial} im Bereich 4 · 10⁻⁶ /K bis 10,6 · 10⁻⁶ /K übt der Grundkörper nach Erkalten einen Druck auf das Fixiermaterial, insbesondere das Glasmaterial, aus und es wird eine Druckeinglasung zur Verfügung gestellt. Andererseits wird durch das Erwärmen aber auch die mechanische Stabilität des Metallstiftes beeinflusst. So erweichen die Metallstifte im allgemeinen bei Temperatureinwirkung. Die Erfinder haben erkannt, dass in der Anwendung für Durchführungen Edelstahl bei Temperatureinwirkung nicht so stark erweicht wie NiFe. Edelmetallstifte bleiben wesentlich biegesteifer als die in derzeitigen Durchführungen verwandten NiFe-Stifte.

Der Massestift 6 wird im dargestellten Fall direkt an der Rückseite des Grundkörpers 1 beispielsweise mittels Lotmaterial 7 befestigt. Üblicherweise handelt es sich um metallisches Lotmaterial. Auch der Massestift 6 kann, wie der eingeglaste Metallstift 5, erfindungsgemäß aus einem Edelstahl, bevorzugt einem Cr-haltigen Edelstahl bestehen.

Der Grundkörper 1 kann in einer Ausführungsform als Stanzteil ausgeführt sein. Ein Stanzteil liegt vor, wenn wenigstens die Durchgangsöffnung 4, vorzugsweise auch die Endgeometrie des Grundkörpers 1 durch Stanzen erzeugt wird. Gemäß einer Ausführungsform kann auch die die äußere Kontur beschreibende Geometrie, insbesondere der Außenumfang des Grundkörpers 1, durch Ausschneiden, bevorzugt Stanzen erzeugt werden. Das Stanzteil kann entweder in der Geometrie wie es nach dem Stanzvorgang vorliegt weiterverwendet werden oder aber in einem weiteren Arbeitsschritt, der sich vorzugsweise direkt anschließt, umgeformt werden, bspw. geprägt oder tiefgezogen werden. Alternativ ist es auch möglich, den gesamten Grundkörper durch ein Kaltumformverfahren wie in der EP 2 431 703 A1 beschrieben, zu erhalten.

Die zur Aufnahme und Fixierung des Metallstiftes 5 mittels des Glaspfropfens 10 vorgesehene Durchgangsöffnung 10 wird durch einen Ausstanzvorganges in Form eines Loches erzeugt. Nachfolgend wird der Metallstift 5 an der Rückseite 11 des Grundkörpers 1 der Metall-Fixiermaterial-Durchführung zusammen mit dem Glaspfropfen 10 in die Durchgangsöffnung 4 eingeführt und der Metallkörper enthaltend den Glaspfropfen 10 und den Metallstift 5 auf ungefähr 600°C erwärmt, so dass nach einem Abkühlungsvorgang das Metall aufschrumpft und so eine kraftschlüssige Verbindung zwischen Glaspfropfen 10 mit Metallstift 5 und Grundkörper 1 ausgebildet wird, auch Druckeinglasung genannt. Aufgrund des Unterschiedes der thermischen Ausdehnungskoeffizienten von Grundkörper 1 und dem Glasmaterial des Glaspfropfens 10 kann diese Druckeinglasung zur Verfügung gestellt werden.

Denkbar ist auch, das Fixiermaterial 10 in einer alternativen Ausgestaltung im geschmolzenen bzw. fließfähigen Zustand, insbesondere die Glasschmelze von der Vorderseite in die Durchgangsöffnung einzubringen. Während des Erkaltens entsteht dann eine form- und stoffschlüssige Verbindung zwischen dem Außenumfang des Metallstiftes 5 und dem Innenumfang der Durchgangsöffnung 4. Der Grundkörper 1 kann derart gestaltet sein, dass das Verhältnis zwischen der Dicke des Grundkörpers 1 und der maximalsten Ausdehnung der Durchgangsöffnung 4 senkrecht zur Achsrichtung der Durchgangsöffnung 4 im Bereich zwischen einschließlich 0,5 bis 2,5 beträgt.

Es ist zu betonen, dass die Erfindung auch mit Isolationsmaterialien in der Durchgangsöffnung 4 entwickelt werden kann, welche nicht auf Glasmaterialien beruhen.

In Figur 1a ist die Verbauung einer Metall-Fixiermaterial-Durchführung in einem Zünderbauteil, beispielsweise einem Airbag-Zünder, gezeigt. Das Zünderbauteil umfasst neben der Metall-Fixiermaterial-Durchführung, in die der Metallstift 5 eingeglast ist, eine Zünderkappe 2, die den Explosivstoff 25 für das Zünderbauteil, d.h. den Airbag-Zünder aufnimmt. Der Explosivstoff 25 wird durch einen elektrischen Impuls des Brückendrahtes 9 ausgelöst. Der Brückendraht 9 verbindet den eingeglasten Metallstift 5 mit dem auf Masse liegenden Grundkörper 1. In der Regel liegt der Brückendraht 9 entgegen der Zeichnung, welche den Brückendraht 9 schematisch und zur Verdeutlichung zeigt, auf der Oberfläche des Grundkörpers 1 und/oder des Fixiermaterials 10 auf.

In Figur 1a deutlich zu erkennen ist auch der Biegepunkt 50 des eingeglasten Metallstiftes, der zu einem axialen Versatz S des Bereiches des Metallstiftes 5, der in die Durchgangsöffnung eingeglast ist und dem Anschlussbereich des Metallstiftes führt. Der Versatz wird so gewählt, dass die Metallstifte der Metall-Fixiermaterial-Durchführung z.B. in Steckersysteme eingeführt werden können. In der Regel werden die beiden Metallstifte 5, 6 so angeordnet und/oder gebogen, dass in der Gesamtschau eine mittige Anordnung beider Metallstifte 5,6 gegenüber dem Grundkörper 1 vorliegt.

In Figur 1b ist in einem Schnitt die in die Zünderkappe 2 eingesetzte Metall-Fixiermaterial-Durchführung umfassend den Grundkörper 1 mit den Metallstiften 5, 6 gezeigt. Gleiche Bauteile wie in Figur 1a sind mit denselben Bezugsziffern belegt. Im Gegensatz zu der in Fig. 1a dargestellten Ausführungsform weist bei der Ausgestaltung gemäß Figur 1b auch der Massestift 6 einen Biegepunkt 60 auf, so dass ein axialer Versatz des mit dem Grundkörper 1 verbundenen Bereichs des Metallstiftes und dessen Anschlussbereich am gegenüberliegenden Ende vorliegt. Deutlich zu erkennen ist in Fig. 1b der Brückendraht 9 zwischen Grundkörper 1 und Metallstift 5. Im Gegensatz zu dem gestanzten Grundkörper aus Fig. 1a handelt es sich bei dem Grundkörper gemäß Figur 1b um einen kaltumgeformten Grundkörper gemäß der EP 2431703 A1 mit einem Freistellungsbereich 17. Die Öffnung 10 wird auch aus dem kalt umgeformten Grundkörper nach Einbringen des Freistellungsbereiches 17 wie in der EP 2431703 A1 ausgestanzt.

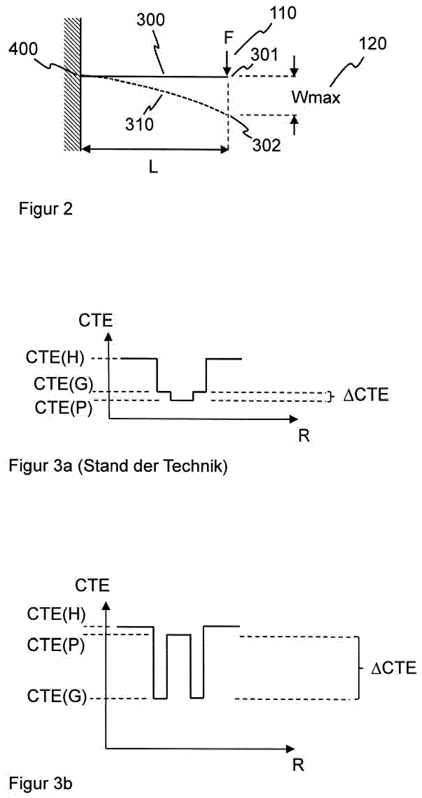

Figur 2 zeigt eine Messordnung bzw. ein Testsystem zur Bestimmung der Biegefestigkeit der Metallstifte. In Figur 2 dargestellt ist ein eingespannter Metallstift 300 mit einer Länge L= 11,68 mm und einem Durchmesser von 1,0 mm auf den eine Kraft F, die mit 110 bezeichnet ist, in Newton (N) einwirkt.

Beim Testsystem bzw. der Messanordnung, wie in Figur 2 gezeigt, bezeichnet 400 die Wand, in die der Stift eingespannt wird, 300 den Stift mit der Länge L sowie 301 den Endpunkt des Metallstiftes im unbelasteten Zustand. Der gebogene, d. h. der belastete Stift wird mit 310 bezeichnet und der Endpunkt des gebogenen Stiftes mit 302. Die Differenz der Endpunkte 301 und 302 bezeichnet die maximale Auslenkung Wₘₐₓ.

Eine Beaufschlagung mit einer Kraft von 6,380 N führt bei einem NiFe-Metallstift im Rohzustand zu einer Durchbiegung von 0,345 mm. Bei höheren Kräften und höherer Durchbiegung geht die elastische Deformation in eine irreversible plastische Deformation über. Im Falle der plastischen Deformation bei Kräften größer 6,380 N und/oder Verbiegungen größer 0,345 mm kann der Stift auch brechen.

Wurde der NiFe-Stift erwärmt, beispielsweise auf eine Einglasungstemperatur von 650°C, so beträgt bei Einwirken einer Kraft auf den Metallstift von nur noch 1,933 N nach dessen Abkühlen (Glühen) die Durchbiegung 0,105 mm. Bei Kräften größer 1,933 N und Durchbiegungen größer 0,105 mm geht in dem geglühten Zustand wie zuvor bzgl. des Rohzustands beschrieben die elastische in eine plastische Verformung über. Diese Betrachtung zu den Kraftbeaufschlagungen zeigt, dass der NiFe-Stift durch die Erwärmung an mechanischer Stabilität stark einbüßt.

Im Gegensatz hierzu tritt bei Verwendung eines Edelstahls, beispielsweise eines ferritischen Edelstahls, insbesondere AISI 430, plastische Verformung erst bei Kräften größer 4,976 N und Biegungen Wₘₐₓ oberhalb von 0,269 mm im Rohzustand und oberhalb von 3,984 N und/oder Biegungen Wₘₐₓ von 0,216 mm im geglühten Zustand auf. Dies zeigt, dass nach einer Wärmebehandlung, beispielsweise mit 650°C, die mechanische Stabilität und/oder die erzielbare maximale Durchbiegbarkeit, des ferritischen Edelstahlstiftes (AISI 430) in dem Testsystem ungefähr 100 % höher ist als bei einem NiFe 47-Stift.

Die angegebenen Werte für Wₘₐₓ repräsentieren in diesen Beispielen die Grenzwerte, bei welchen noch eine elastische Verbiegung des Metallstifts vorliegen. Wir die mechanische Belastung in diesen Beispielen über die genannten Grenzwerte erhöht, erfolgt natürlich eine Verbiegung über die genannten Werte von Wₘₐₓ hinaus, die dann aber eine plastische Verbiegung, d.h. irreversibel ist.

In der Praxis bedeutet das, dass durch den Einglasungsprozess, z.B. bei Temperaturen von 650°C, der Edelstahlstift im Gegensatz zum NiFe 47-Stift, der sehr viel stärker erweicht, deutlich biegesteifer ist. Mit dem Edelstahl wird somit ein Material zur Verfügung gestellt, das es erlaubt, einen Metallstift derart auszulegen, dass er im post-erhitzten, d.h. geglühten Zustand in einem Testsystem mit der Metallstiftlänge L von 11,68 mm beim senkrechten Beaufschlagen am Endpunkt erst bei einer Kraft Fₘₐₓ größer 2,5 N, vorteilhaft größer 3 N, besonders vorteilhaft größer 3,2 N oder 3,5 N, in diesem Beispiel 3,984 N, plastisch verformt.

Ebenso bedeutet das, dass die maximale elastische Biegbarkeit und/oder Auslenkung Wₘₐₓ in dem genannten Testsystem vorteilhaft mehr als als 0,15 mm beträgt, insbesondere im Bereich von 0,15 mm bis 0,3 mm und/oder 0,4 mm liegt.

Dadurch wird die Wahrscheinlichkeit, dass der Metallstift bei mechanischer Belastung in der Nachbearbeitung beschädigt wird, insbesondere bei der Beaufschlagung mit Biegekräften in der Assemblierung, durch die Erfindung deutlich reduziert.

In den Figuren 3a und 3b ist der Unterschied der Ausdehnungskoeffizienten bei herkömmlichen Durchführungen und bei der erfindungsgemäßen Durchführung gezeigt.

Figur 3a zeigt die Ausdehnungskoeffizienten bei herkömmlichen Metall-Fixiermaterial-Durchführungen. Hierbei bezeichnet CTE (H) den Ausdehnungskoeffizienten a_{Grundkörper} bzw. des Headers, CTE (G) den Ausdehnungskoeffizienten des Fixiermaterials α_{Glas} und CTE (P) den Ausdehnungskoeffizienten des Metallstifts α_{Metallstift}, der im Fixiermaterial angeordnet ist. Wie aus Figur 3a hervorgeht ist, um eine Druckeinglasung zur Verfügung zu stellen, der Ausdehnungskoeffizient des Grundkörpers (CTE (H)) deutlich größer als der Ausdehnungskoeffizient (CTE (G)) des Fixiermaterials, insbesondere des Glases. Beispielsweise liegt der Ausdehnungskoeffizient CTE (H) des Grundkörpers im Bereich von 18,3 * 10⁻⁶ / K im Falle von austenitischem Edelstahl als Material des Grundkörpers. Der Ausdehnungskoeffizient des Glasmaterials (CTE (G)), der auch mit α_{Glas} bezeichnet wird, liegt üblicherweise im Bereich 4 * 10⁻⁶ / K bis 10,6 * 10⁻⁶/K und damit deutlich unter dem Ausdehnungskoeffizienten CTE (H) des Grundkörpers. Im Stand der Technik war es so, dass der Ausdehnungskoeffizient CTE (P) des Metallstiftes stets geringer war als der des umgebenden Glasmaterials, wenn auch nur geringfügig. Bisher ging man davon aus, dass dies erforderlich ist um eine dauerhafte Fixiermaterial-Durchführung zu erreichen, weil sich ansonsten bei thermischen Schwankungen der Metallstift von dem Glas ablösen könnte. Daher werden bisher insbesondere Metallstifte aus nicht-Edelstählen, v.a. NiFe, für diese Anwendung eingesetzt.

Figur 3b zeigt die Ausdehnungskoeffizienten bei einer erfindungsgemäßen Durchführung mit einem Metallstift aus Edelstahl. Wie aus Figur 3b hervorgeht, ist der Ausdehnungskoeffizient des Metallstiftes (CTE (P)) zwar geringer als der des Grundkörpers (CTE (H)), aber höher als der Ausdehnungskoeffizient CTE (G) des Fixiermaterials. Während die Edelstähle Ausdehnungskoeffizienten im Bereich 11,0 bis 13,5 * 10⁻⁶ /K aufweisen, liegt der Ausdehnungskoeffizient des Fixiermaterials, z.B. des Glases üblicherweise lediglich im Bereich 4 * 10⁻⁶ / K bis 10,6 * 10⁻⁶/K, insbesondere 6,1 * 10⁻⁶ /K bis 10,6 * 10⁻⁶ /K und damit unterhalb dem Ausdehnungskoeffizienten des Metallstiftes. Obwohl somit der Ausdehnungskoeffizient des Metallstiftes größer ist als derjenige des Glasmaterials, entgegen dem Stand der Technik wie in Figur 3a abgebildet, kann auch für den erfindungsgemäßen Metallstift aus Edelstahl mit α_{Metallstift} > α_{Glas} eine ausreichende Dichtheit und eine Druckeinglasung zur Verfügung gestellt werden, wenn ein positiver Fugendruck vom Grundkörper mit dem Ausdehnungskoeffizienten α_{Grundkörper} bzw. CTE (H) auf das Glas ausgeübt wird. Bei einem hohen Fugendruck, der vom Grundkörper auf das Glasmaterial und den Metallstift ausgeübt wird, bleibt der Übergang zwischen Glas und Metall, insbesondere der Übergang vom Glas zum Metallstift geschlossen und Dichtheit (wird gewährleistet. Insbesondere kann auch eine hermetische Dichtheit erreicht werden. Ein ausreichend hoher Fugendruck kann vorteilhaft erreicht werden, wenn in der Aufsicht die Fläche des Grundkörpers abzüglich der Fläche der Durchgangsöffnung mindestens das 1,2-fache der Fläche der Durchgangsöffnung entspricht.

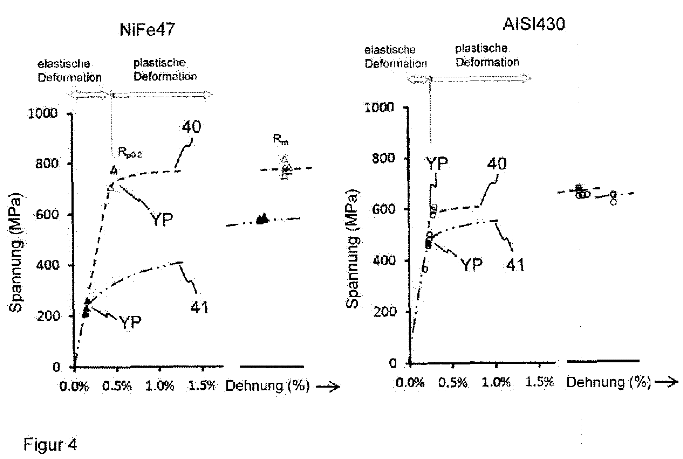

In Figur 4 ist die Spannungs (Stress) - Dehnungs (Strain)-Kurve für einen erfindungsgemäßen Edelstahlstift und im Vergleich hierzu einem NiFe-Stift (NiFe 47) gezeigt. Wie aus Figur 4 deutlich hervorgeht, büßt der NiFe-Stift gegenüber dem Edelstahlstift (AlSI430), insbesondere dem aus einem ferritischen Edelstahl, nach Erwärmen, z.B. auf 650 °C wie sie für das Einglasen notwendig ist, stark an Stabilität ein und wird geschwächt. So verschiebt sich beim ferritischen Edelstahl AISI 430 der Übergangspunkt von der elastischen Deformation zur plastischen Deformation bei ungefähr 0,25 % Dehnung lediglich von einer Spannung von etwa 600 MPa für das Rohmaterial auf 500 MPa für das geglühte Material, d.h. die Spannung am Übergangspunkt sinkt nur um ungefähr 20 % ab. Im Gegensatz hierzu verschiebt sich der Übergangspunkt von der elastischen zur plastischen Verformung bei Erwärmung im Fall des NiFe-Metallstiftes bei ungefähr 0,25 % Dehnung von einer Spannung von 700 MPa zu 200 MPa, d.h. der Übergangspunkt des Rohmaterials ist 3,5 mal höher als der des geglühten Materials. Dies zeigt die Überlegenheit des Edelstahlmaterials gegenüber NiFe als Stiftmaterial für Systeme, in denen das Metallstift in einem thermisch behandelten, insbesondere geglühten Zustand voriegt. Dies ist umso bemerkenswerter, da die Festigkeit des NiFe-Metallstifts im unbehandelten, d.h. nicht wärmebehandelten Zustand durchaus höher ist als die des Edelstahlstifts, so dass man davon ausgehen sollte, dass die bisherigen nicht-Edelstahlstifte, neben der beschriebenen passenderen thermischen Ausdehnung, auch aus diesem Grund geeigneter zu sein schienen.

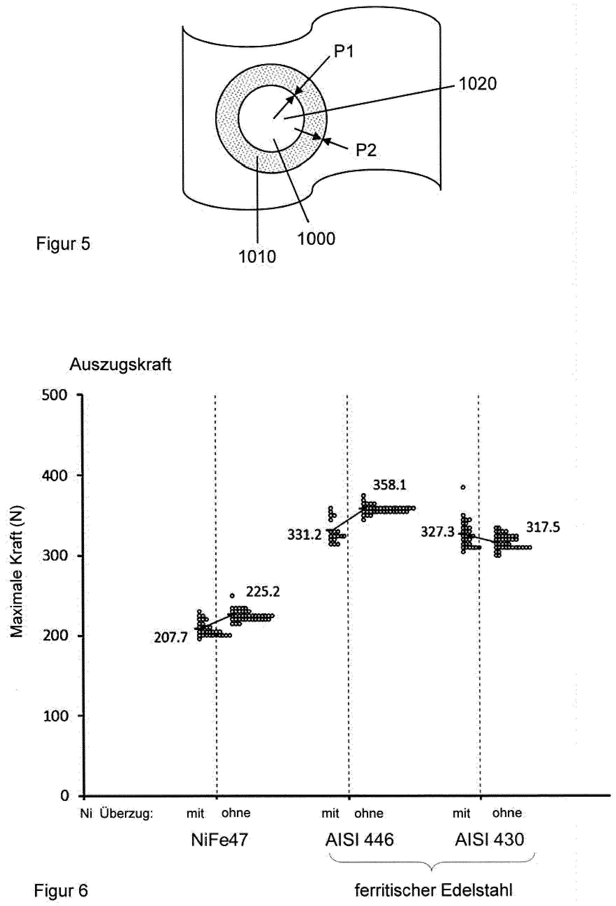

Figur 5 zeigt ein Gehäusebauteil in der Draufsicht, wobei das Gehäusebauteil eine Öffnung 1000 umfasst, in die in einem Glasmaterial 1010 ein Stift 1020 eingeglast ist. Ebenfalls in Figur 5 eingezeichnet ist der Fugendruck P1 des Glasmaterials auf den Metallstift 1020 und der Fugendruck P2 des Grundkörpers bzw. Gehäusebauteils auf das Glasmaterial. Erfindungsgemäß muss für den Fall, dass der Ausdehnungskoeffizient des Metallstiftes größer als der des Glasmaterials ist, für eine ausreichende Dichtheit eine ausreichende Druckvorspannung des Grundkörpers bzw. Gehäusebauteils auf das Glas aufgeprägt werden. Dies wird insbesondere erreicht, wenn die zuvor beschriebene Geometrievorschrift eingehalten wird.

Aus Figur 6 geht hervor, dass überraschenderweise bei einem Edelstahlstift gegenüber einem bisher verwendeten Metallstift aus nickelhaltigen Eisenmaterial (NiFe 47) um bis zum 50 % größere Auszugskräfte durch die Verwendung eines Edelstahlmaterials erreicht werden können. Wie Figur 6 zeigt, betragen die

Auszugskräfte für NiFe 47 lediglich 207,7 N für ein nicht beschichtetes Material und für einen mit Nickel beschichteten NiFe 47 Stift 225,2 N.

Überraschenderweise können mit dem erfindungsgemäßen Metallstift aus einem Edelstahlmaterial, insbesondere des ferritischen Edelstahles, wesentlich höhere Auszugskräfte erreicht werden. Dies ist insbesondere überraschend, da wie dargelegt die Lage der thermischen Ausdehnungskoeffizienten für den Edelstahlstift eher ungünstig sind. Für das ferritische Edelstahlmaterial AISI 446 werden ohne einen Nickelüberzug Auszugskräfte von 331,2 N erreicht, mit einem Nickelüberzug Auszugskräfte von 358,1N. Etwas niedriger liegen die Auszugskräfte beim Edelstahl AISI 430. Dort beträgt die Auszugskraft ohne einen Nickelüberzug 317,5 N und mit einem Nickelüberzug 327,3 N.

Dies zeigt, dass die Metallstifte aus Edelstahl sich gegenüber herkömmlichen NiFe-Stiften nicht nur durch eine höhere mechanische Festigkeit, sondern durch höhere Auszugskräfte auszeichnen. Es kann vermutet werden, dass die verbesserten Auszugskräfte des Metallstifts aus Edelstahl daher beruhen, dass das Material des Metallstifts nach Erwärmen härter bleibt als das des NiFe-Stifts, so dass der Edelstahl dem Fugendruck, den der Header über das Glas auf den Metallstift überträgt, mehr Kraft entgegensetzen kann und sozusagen weniger eingedrückt wird.

Der Nickelüberzug oder der Goldüberzug oder ein Goldüberzug auf einem Nickelüberzug auf einem oder den Metallstiften dient neben dem zur Verfügung stellen hoher Auszugskräfte dazu, dass die Metallstifte einfach kontaktiert werden können.

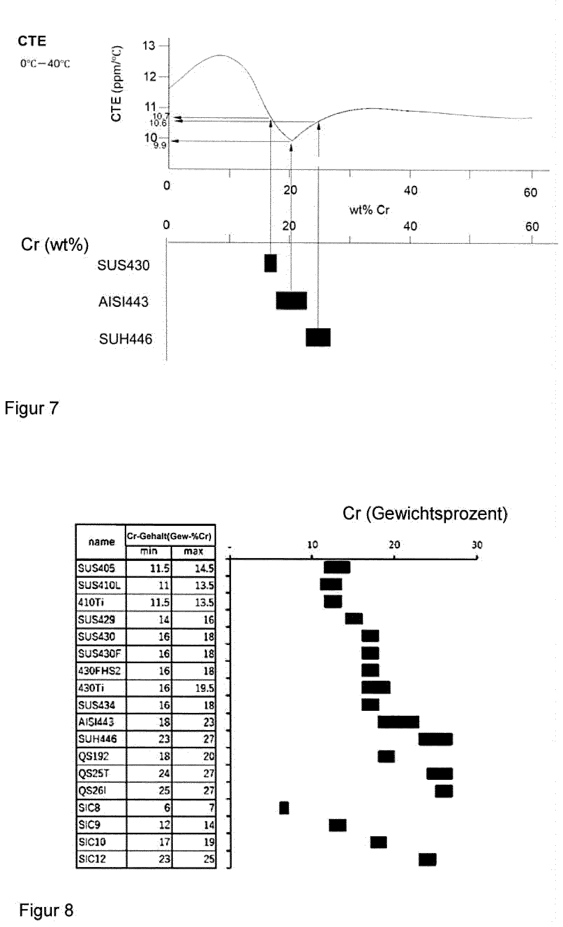

Figur 7 zeigt den Effekt des Chromanteils des Edelstahls auf den thermischen Ausdehnungskoeffizienten α bzw. CTE (P) von Edelstahl, wie er beim Metallstift verwandt wird. Angegeben ist der lineare thermische Ausdehnungskoeffizient von 0-40°C in ppm/°C, d. h. * 10⁻⁶/K, für einen Chromanteil von 0-60 Gewichtsprozent. Wie aus Figur 7 hervorgeht, wird für einen Chromanteil von ungefähr 20 Gewichtsprozent für einen Edelstahl AISI 443 ein thermischer Ausdehnungskoeffizient von 9,9 * 10⁻⁶ /K erreicht. Generell ist zu sehen, dass der CTE von dem Chromanteil abhängig ist. Anhand von Figur 7 ist ersichtlich, dass ein lokales Minimum des CTEs bei ungefähr 20 Gew.% Chromanteil erreicht wird. Ein besonders vorteilhafter Edelstahl für den Metallstift wird also so gewählt, dass dessen Chromanteil im Bereich um das lokale Minimums des CTEs liegt, insbesondere im Bereich von 10 Gewichtsprozent Chromanteil bis 30 Gewichtsprozent, besonders vorteilhaft von 14 Gewichtsprozent bis 28 Gewichtsprozent Chromanteil. Die Edelstähle SUS430, AlSI443 und SUH446 liegen in diesem Bereich und sind besonders vorteilhaft als Material des Metallstifts verwendbar. Gleiches gilt für AISI 446 und AISI 430.

Wie später ausgeführt werden wird, haben Materialien mit diesen Chromanteilen bzw. Chrom-Äquivalentanteilen auch Vorteile bzgl. des elektrochemischen Potentials.

Figur 8 zeigt den Chromgehalt bei Edelstählen.

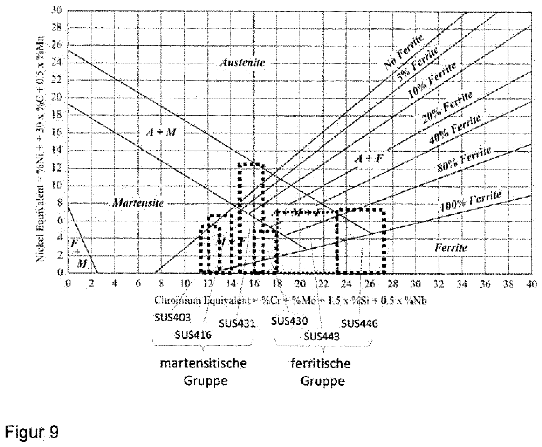

In Figur 9 dargestellt ist das Chrom- und das Nickeläquivalent für martensitische und austinitische Edelstähle.

Das Chromäquivalent, das neben Chrom auch noch den Anteil an Molybdän sowie Silizium und Niob berücksichtigt, ist sowohl für martensitische Edelstähle als auch für ferritische Edelstähle angegeben. Das Chromäquivalent liegt im Bereich 10 bis 30 Gewichtsprozent, insbesondere 12 Gewichtsprozent bis 28 Gewichtsprozent. Besonders vorteilhafte Bereiche im Sinne der Erfindung sind in der Figur 8 durch durchbrochene Linien markiert.

Generell kann gesagt werden, dass ein erfindungsgemäßer Edelstahl einen chromlegierter Stahl umfasst oder ist oder ein Chromäquivalent aufweist, wobei das Chromäquivalent % Cr + % Mo + 1,5 * %Si + 0,5 * % Nb ist. Das Chromäquivalent gibt gebräuchlicherweise eine Maßzahl für die Gesamtheit der ferritbildenden Elemente einer austenitischen Edelstahllegierung an, nach der empirischen Formel von Schaeffler und DeLong. Besonders bevorzugte Bereiche der Chromäquivalente sind die in Figur 8 umrandeten Bereiche.

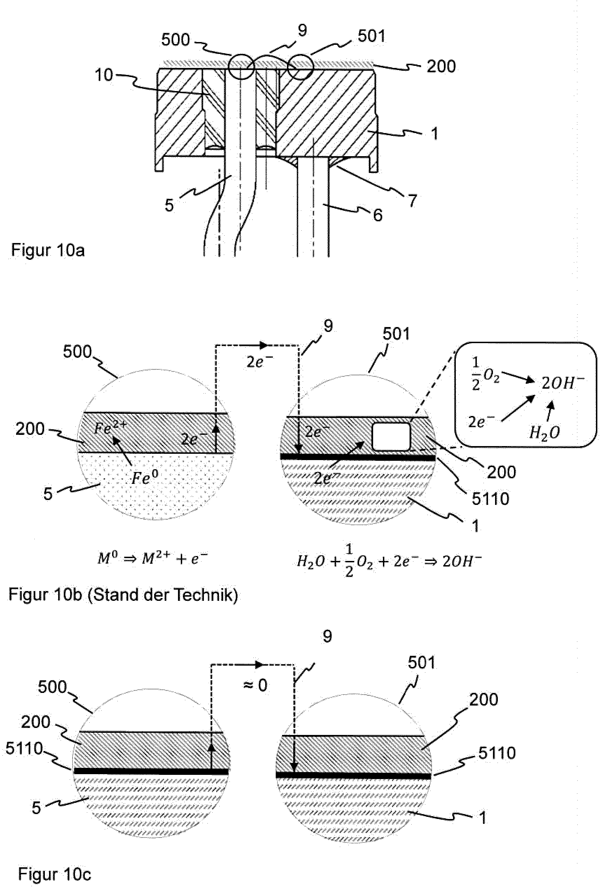

Figur 10a zeigt das Kopfteil einer Metall-Fixiermaterial-Durchführung gemäß der Erfindung und Figur 10b und 10c das Schema der elektrochemischen Reaktionen im Bereich der Metall-Fixiermaterial-Durchführung aufgrund unterschiedlicher elektrochemischer Potentiale im Stand der Technik und gemäß der Erfindung.

Figur 10a zeigt zunächst den Kopfteil einer erfindungsgemäßen Glas-Metall-Fixiermaterial-Durchführung, wie in den Figuren 1a und 1b gezeigt. Gleiche Bauteile wie in den Figuren 1a und 1b sind mit denselben Bezugsziffern belegt. Wie aus Figur 10a hervorgeht, kann sich auf der Oberfläche des Grundkörpers 1 ein leitender Film, beispielsweise ein Wasserfilm 200, ausbilden, so dass es bei unterschiedlichem elektrochemischem Potential des Grundkörpers und des Metallstiftes zu einem Elektronenfluss vom Metallstift zur Masse, hier dem Grundkörper 1, kommt, wodurch eine Oxidation des Metallstiftes und/oder des Grundkörpers und/oder sogar des Fixiermaterials, z.B. dem Glasmaterial, hervorgerufen werden kann. Der Elektronenfluss vom Metallstift zum Grundkörper erfolgt über den Brückendraht 9. Der elektrisch leitende Film 200 kann insbesondere im Langzeitbetrieb einer gattungsgemäßen Durchführung vorhanden sein und eine Rolle für die Langzeitstabilität der und Korrossionangriffe auf die Durchführung spielen. Die Elektronen fließen über den Brückendraht 9 vom Kopf des Metallstiftes 500 zum Grundkörper 501 oder ggfls. in die andere Richtung, je nach Potentialdifferenz. Das isolierende Material zwischen Grundkörper und Metallstift ist in der Regel ein nicht leitendes Fixiermaterial, bevorzugt ein Glas- oder Glaskeramikmaterial. Das Glas- bzw. Glaskeramikmaterial, in das der metallische Leiter eingeglast ist, ist mit 10 bezeichnet. Der durch die Öffnung hindurch geführte Metallstift trägt die Bezugsziffer 5, der am Grundkörper mit Hilfe eines Lotmaterials 7 angelötete Metallstift die Bezugsziffer 6.

In Figur 10b ist der Elektronenfluss vom Metallstift zum Grundkörper aufgrund unterschiedlicher elektrochemischer Potentiale dargestellt. Dies entspricht dem Stand der Technik. So betrug der Unterschied im elektrochemischen Potential von Metallstift aus einem Nicht-Edelstahl, insbesondere NiFe, und Grundkörper im Stand der Technik mehr als 0,3 V. Aufgrund dieses Unterschiedes im elektrochemischen Potential kann es bei Vorliegen einer elektrischen leitfähigen Schicht auf dem Metallstift und dem Grundkörper, beispielsweise bei Vorliegen eines Wasserfilms 200, zu einer Umsetzung von Eisen zu Fe²⁺ und Abgabe von zwei Elektronen kommen, die aufgrund der bestehenden elektrischen Verbindung zum Grundkörper gewandert sind. Mit Sauerstoff entstehen bei Anwesenheit von Wasser 2 OH⁻ wie in Figur 10b dargestellt, das heißt der Wasserfilm wird zunehmend basisch und das Material des Metallstiftes oxidiert zu Fe²⁺. Gleiche Bezugsziffern wie in Figur 10a sind auch in Figur 10b identisch belegt. So bezeichnet 500 den Bereich des Metallstiftes, 501 den Bereich des Grundkörpers. Der Elektronenfluss vom Metallstift zum Grundkörper erfolgt über den Brückendraht 9 oder sogar über den elektrisch leitenden Film 200, insbesondere wenn dieser bei fortschreitender Reaktion immer basischer wird. Die zunehmende Basizität des Films 200 kann den Korrosionsangriff auf die Metalle und sogar das Glasmaterial verstärken. Die Schicht 5110 auf dem Grundkörper aus Edelstahl ist eine Passivierungsschicht, die sich auf dem Edelstahl bildet und insbesondere Sauerstoff enthalten kann. Bei NiFe als Stiftmaterial wurde beobachtet, dass sich eine lokale Zelle zwischen dieser Passivierungsschicht 5110 und dem Metallstift aus Nicht-Edelstahl bilden kann, insbes. solchen aus NiFe. Die elektrochemische Potentialdifferenz zwischen NiFe als Stiftmaterial und z.B. AISI 304L als Material des Grundkörpers liegt bei 0,38 V. Hierbei kann es zu einer elektrochemischen Korrosion kommen.

Während im Stand der Technik somit der Metallstift korrodieren kann, ist dies gemäß der Erfindung nicht mehr möglich oder zumindest stark unterdrückt, da der Metallstift im Bereich 500 im Wesentlichen das gleiche elektrochemische Potential wie der Grundkörper mit der Bezugsziffer 501 aufweist. Wie in Figur 10c dargestellt, findet dann kein Elektronenfluss vom Metallstift zum Grundkörper statt, selbst wenn Grundkörper und Metallstift mit einem elektrisch leitenden Film 200, insbesondere einem Wasserfilm versehen sind. Die Oberfläche 5110 von Grundkörper und insbesondere Metallstift sind sich auf Edelstahl bildende Passivierungsschichten, die Sauerstoff enthalten können, aber nicht weiter oxidieren. Ebenso entsteht insbesondere kein zunehmend basischer werdender Wasserfilm.

Bei AISI 430 als Material des eingeglasten Metallstifts und AISI 304 als Material des Grundkörpers liegt der Absolutbetrag der elektrochemische Potentialdifferenz bei 0,02 V. Hierbei sind elektrochemische Korrosionsangriffe zumindest sehr stark unterdrückt.

Gleiche Bauteile wie in den vorangegangen Figuren sind mit denselben Bezugsziffern belegt. Bevorzugt beträgt der Absolutbetrag des Unterschieds im elektrochemischen Potential von Grundkörper und Edelstahlstift bei dem erfindungsgemäßen Bauteil lediglich von 0,3 V bis 0,0 V, vorteilhaft 0,1 V bis 0,0 V, besonders vorteilhaft 0,05 V bis 0,0 V.

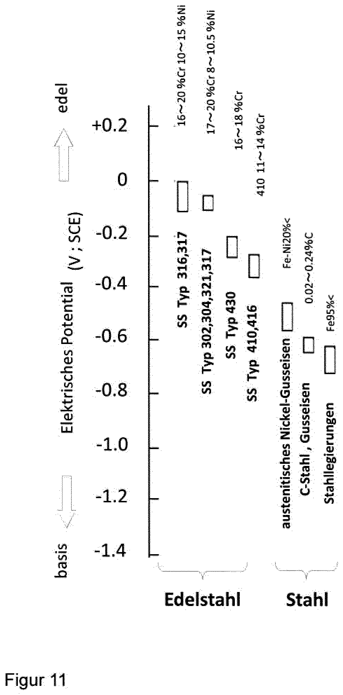

Figur 11 zeigt die Lage der elektrochemischen Potentiale einer Auswahl von möglichen Materialien, insbesondere von Edelstählen für den oder die Metallstifte und/oder aber auch für den Grundkörper. Wie beschrieben kommt es zum Erzielen einer guten Korrisionsbeständigkeit auf eine entsprechende Materialkombination von Grundkörper und Metallstift in dem Fixiermaterial an. Bei der Auswahl wird angestrebt, dass sich eine möglichst geringe Potentialdifferenz ergibt. Das Material des Grundkörpers muss allerdings auch andere Anforderungen erfüllen. Insbesondere muss es mit der Metallkappe 2 verschweißbar sein, insbesondere durch Laserschweißen. Ebenso ist das Herstellungsverfahren des Grundkörpers ein Aspekt, insbesondere wenn dieser ausgestanzt oder kaltgeformt wird. Bei kaltgeformten Grundkörpern kann ein Kupferanteil vorteilhaft sein.

Bei der Auswahl der Metallstifte 5,6, insbesondere des in dem Fixiermaterial angeordneten Metallstifts 5, und auch für die Auswahl des Materials des Grundkörpers sind die in der Figur 11 als Edelstahl (stainless steel) aufgeführten Materialien besonders vorteilhaft. Dies sind die Edelstahltypen (AISI) 316, 317, 302, 304, 321, 317, 430, 410 und/oder 416. Sie zeichnen sich allesamt dadurch aus, dass sie eine geringe elektrochemische Potentialdifferenz zu Meerwasser aufweisen, insbesondere einen Absolutbetrag von weniger als 0,4 V, vorteilhaft weniger als 0,36 V, was wie beschrieben ein gutes Maß für die Beurteilung der Resistenz gegenüber galvanischen Korrosionsangriffen für die gesamte Durchführung ist.

Mit der Erfindung wird somit erstmals eine Metall-Fixiermaterial-Durchführung angegeben, die sich zum einen durch eine höhere mechanische Stabilität insbesondere bei Verbiegung der Metallstifte und/oder höhere Auszugskräfte der Metallstifte auszeichnet und vorteilhaft auch durch eine geringere Korrosionsanfälligkeit, insbesondere in widrigen Anwendungsbedingungen.

Durch die verbesserte mechanische Stabilität können Assemblierungsfehler reduziert werden, was sich in einer verbesserten Zuverlässigkeit und/oder geringerem Ausschuss niederlegt. Die verbesserten Korrosionsbeständigkeit trägt zur Langzeitstabilität und damit Sicherheit der Einrichtungen bei, in welchen die erfindungsgemäße Durchführung eingesetzt wird. Insgesamt kann so die Effizienz der Herstellung von Gegenständen enthaltend die erfindungsgemäße Durchführung sowie deren Sicherheit erhöht werden.

## Patentansprüche

1. Metall-Fixiermaterial-Durchführung für Anzünder von Airbags und/oder Gurtstraffern, mit zumindest einem Metallstift (5), der in eine Durchgangsöffnung (4) eines Grundköpers (1) in einem glasigen oder glaskeramischen Fixiermaterial (10) eingeschmolzen ist,
wobei der wenigstens eine Metallstift (5) zumindest in seinem Kernbereich aus einem Edelstahl nach EN 10020:2000 besteht,
**dadurch gekennzeichnet, dass** der Edelstahl ein ferritischer Edelstahl ist und derart gewählt ist, dass der zumindest eine Metallstift (5) umgerechnet auf die Standarddimensionierung eines Metallstiftdurchmessers von 1,00 ± 0,03 mm und einer Metallstiftlänge von 11,68 ± 0,02 mm eine maximale elastische Verbiegung Wₘₐₓ von zumindest 0,13 mm aufweist, bevorzugt zumindest 0,15 mm, besonders bevorzugt zumindest 0,18 mm, ganz besonders bevorzugt zumindest 0,24 mm.

2. Metall-Fixiermaterial-Durchführung (1) nach Anspruch 1,
**dadurch gekennzeichnet, dass**
der Edelstahl derart gewählt ist, dass bei einer mechanischen Belastung im Bereich 3N bis 4N eine Auslenkung des zumindest einen Metallstifts (5) von höchstens 0,21 mm erfolgt, insbesondere eine elastische Auslenkung.

3. Metall-Fixiermaterial-Durchführung (1) nach mindestens einem der vorhergehenden Ansprüche,
**dadurch gekennzeichnet, dass**
die mechanische Belastung von 0,25% Dehnung des zumindest eines Metallstifts (5) einer Spannung von mehr als 450 MPa entspricht, bevorzugt mehr als 480 MPa oder mehr als 500 MPa, insbesondere bevorzugt von 450 MPa bis 700 MPa.

4. Metall-Fixiermaterial-Durchführung (1) nach mindestens einem der vorhergehenden Ansprüche,
**dadurch gekennzeichnet, dass** die Härte des zumindest einen Metallstifts (5) in einem geglühten Zustand derart gewählt ist, dass
die Auszugskraft des zumindest einen Metallstiftes (5) aus dem Glasmaterial der Durchgangsöffnung (11) mehr als 250 N beträgt, insbesondere von 250 N bis 400 N, bevorzugt 300 N bis 380 N.

5. Metall-Fixiermaterial-Durchführung (1) nach mindestens einem der vorhergehenden Ansprüche,
**dadurch gekennzeichnet, dass**
der Grundkörper (1) aus einem Metall, insbesondere Stahl, rostfreier Stahl, Edelstahl, Titan, einer Titanlegierung, Magnesium, einer Magnesiumlegierung, eine Aluminiumlegierung, Aluminium oder AlSiC besteht und/oder dieses im Wesentlichen umfasst.

6. Metall-Fixiermaterial-Durchführung (1) nach dem vorhergehenden Anspruch, **dadurch gekennzeichnet, dass**
der Edelstahl des Grundkörpers (1) ein austenitischer Edelstahl ist.

7. Metall-Fixiermaterial-Durchführung (1) nach mindestens einem der vorhergehenden Ansprüche,
**dadurch gekennzeichnet, dass**
der Edelstahl des wenigstens einen Metallstiftes (5) derart ausgewählt ist, dass der thermische Ausdehnungskoeffizient α_{Metallstift} bei einer Temperatur von 650°C im Bereich von 9 bis 15, bevorzugt von 11,0 • 10⁻⁶ /K bis 14,0 • 10⁻⁶ /K, bevorzugt 11,5 •10⁻⁶ /K bis 14,0 • 10 ⁻⁶ 1/K, insbesondere 11•10⁻⁶ /K bis 13,5• 10 ⁻⁶ 1 /K, besonders bevorzugt 11,5 • 10⁻⁶ /K bis 12,5 • 10 ⁻⁶ 1/K liegt.

8. Metall-Fixiermaterial-Durchführung nach mindestens einem der vorangehenden Ansprüche,
**dadurch gekennzeichnet, dass**
das glasige oder glaskeramische Fixiermaterial (10) einen thermischen Ausdehnungskoeffizient a_{Glas} bei einer Temperatur bis zu Tg des Fixiermaterials im Bereich 4 •10 ⁻⁶ 1/K bis 10,6 • 10 ⁻⁶ 1/K aufweist.

9. Metall-Fixiermaterial-Durchführung nach mindestens einem der vorangehenden Ansprüche,
**dadurch gekennzeichnet, dass**
der Grundkörper einen thermischen Ausdehnungskoeffizient α_{Grundkörper} aufweist, der mindestens 2 • 10 ⁻⁶ 1/K, bevorzugt 10 • 10 ⁻⁶ 1/K höher ist als der thermische Ausdehnungskoeffizient α_{Glas} des Glases, bevorzugt im Bereich 11 • 10 ⁻⁶ 1/K bis 18 • 10 ⁻⁶ 1/K liegt.

10. Metall-Fixiermaterial-Durchführung (1) nach mindestens einem der vorhergehenden Ansprüche,
**dadurch gekennzeichnet, dass**
der zumindest eine Metallstift (5) zumindest einen Biegepunkt (50) aufweist, wobei der Metallstift (5) bevorzugt so gebogen ist, dass ein axialer Versatz S des Bereichs des Metallstifts (5) in der Durchgangsöffnung (4) und des Anschlussbereichs an dessen gegenüberliegenden Ende vorliegt

11. Metall-Fixiermaterial-Durchführung (1) nach mindestens einem der vorhergehenden Ansprüche,
**dadurch gekennzeichnet, dass**
zumindest ein weiterer Metallstift (6) elektrisch leitfähig mit dem Grundkörper (1) verbunden ist, insbesondere mittels einer Lotverbindung (7) oder einer Schweißverbindung.

12. Metall-Fixiermaterial-Durchführung (1) nach Anspruch 11,
**dadurch gekennzeichnet, dass**
der elektrisch leitfähig mit dem Grundkörper verbundene weitere Metallstift (6) zumindest in seinem Kernbereich aus einem Nicht-Edelstahl besteht, insbesondere aus NiFe, und wobei dieser Metallstift (6) mittels einer Schweißverbindung mit dem Grundkörper verbunden ist, und dieser Metallstift (6) bevorzugt in einem nicht geglühten Zustand vorliegt.

13. Metall-Fixiermaterial-Durchführung (1) nach Anspruch 11,
**dadurch gekennzeichnet, dass**
der weitere Metallstift (6) zumindest einen Biegepunkt (60) aufweist, wobei dieser weitere Metallstift (6) bevorzugt so gebogen ist, dass ein axialer Versatz des mit dem Grundkörper verbundenen Bereichs des Metallstifts (6) und des Anschlussbereichs an dessen gegenüberliegenden Ende vorliegt

14. Metall-Fixiermaterial-Durchführung (1) nach mindestens einem der vorhergehenden Ansprüche,
**dadurch gekennzeichnet, dass**
der zumindest eine in der Durchgangsöffnung (4) eingeglaste Metallstift (5) und/oder der mit dem Grundkörper (1) elektrisch leitfähig verbindende Metallstift (6) mit Nickel beschichtet ist; wobei die Nickelschicht bevorzugt in Bereichen der Metallstifte (5,6) vorliegt, welche ausgewählt sind aus der Gruppe einschließlich Kombinationen derselben:
- am eingeglasten Metallstift (5) in einem Bereich, der mit einer Goldschicht auf zumindest Bereichen der Nickelschicht versehen ist, insbesondere in dem Anschlussbereich am Ende des Metallstifts (5),
- am eingeglasten Metallstift (5) in einem Bereich, der mit dem Fixiermaterial in Kontakt steht,
- am elektrisch leitfähig mit dem Grundkörper verbundenen Metallstift (6) in einem Bereich, der mit einer Goldschicht auf zumindest Bereichen der Nickelschicht versehen ist, insbesondere in dem Anschlussbereich am Ende des Metallstifts (5),
- am elektrisch leitfähig mit dem Grundkörper verbundenen Metallstift (6) in einem Bereich, an dem der Metallstift (6) mittels einem metallischen Lotmaterial (7) mit dem Grundkörper verbunden ist.

15. Metall-Fixiermaterial-Durchführung (1) nach mindestens einem der vorhergehenden Ansprüche,
**dadurch gekennzeichnet, dass**
der zumindest eine in der Durchgangsöffnung (4) eingeglaste Metallstift (5) und/oder der mit dem Grundkörper (1) elektrisch leitfähig verbundene weitere Metallstift (6) mit Gold beschichtet ist, wobei die Goldschicht bevorzugt zumindest im Anschlussbereich des Metallstifts (5) und/oder des elektrisch leitfähig mit dem Grundkörper verbundenen weiteren Metallstifts (6) vorliegt, welcher dem im und/oder am Grundkörper befindlichen Ende des jeweiligen Metallstifts gegenüber liegt.

## Claims

1. Metal-fixing-material leadthrough for igniters of airbags and/or belt tensioners, having at least one metal pin (5) which is fused into a through-opening (4) of a base body (1) in a vitreous or glass-ceramic fixing material (10), wherein the at least one metal pin (5) consists, at least in its core region, of a stainless steel according to EN 10020:2000,
**characterized in that**
the stainless steel is a ferritic stainless steel and is selected such that the at least one metal pin (5), converted to the standard dimensioning of a metal pin diameter of 1.00 ±0.03 mm and a metal pin length of 11.68 ±0.02 mm, has a maximum elastic bending Wₘₐₓ of at least 0.13 mm, preferably at least 0.15 mm, particularly preferably at least 0.18 mm, more particularly preferably at least 0.24 mm.

2. Metal-fixing-material leadthrough (1) according to claim 1,
**characterized in that**
the stainless steel is selected in such a way that a deflection of the at least one metal pin (5) of at most 0.21 mm occurs, in particular an elastic deflection, in the event of a mechanical load in the range 3N to 4N.

3. Metal-fixing material leadthrough (1) according to at least one of the preceding claims,
**characterized in that**
the mechanical load of 0.25% elongation of the at least one metal pin (5) corresponds to a tension of more than 450 MPa, preferably more than 480 MPa or more than 500 MPa, in particular preferably from 450 MPa to 700 MPa.

4. Metal-fixing material leadthrough (1) according to at least one of the preceding claims,
**characterized in that**
the hardness of the at least one metal pin (5) in an annealed state is selected such that the pull-out force of the at least one metal pin (5) from the glass material of the through-opening (11) is more than 250 N, in particular from 250 N to 400 N, preferably 300 N to 380 N.

5. Metal-fixing-material leadthrough (1) according to at least one of the preceding claims,
**characterized in that**
the base body (1) consists of a metal, in particular steel, stainless steel, high-grade steel, titanium, a titanium alloy, magnesium, a magnesium alloy, an aluminum alloy, aluminum or AlSiC.

6. Metal-fixing-material leadthrough (1) according to the preceding claim, **characterized in that**
the stainless steel of the base body (1) is an austenitic stainless steel.

7. Metal-fixing-material leadthrough (1) according to at least one of the preceding claims,
**characterized in that**
the stainless steel of the at least one metal pin (5) is selected such that the coefficient of thermal expansion a_{metal pin} at a temperature of 650°C is in the range of from 9 to 15, preferably from 11.0 • 10⁻⁶ /K to 14.0 • 10⁻⁶ /K, preferably 11.5 • 10⁻⁶ /K to 14.0 • 10⁻⁶ 1/K, in particular 11 • 10⁻⁶ /K to 13.5 • 10⁻⁶ 1/K, particularly preferably 11.5 • 10⁻⁶ /K to 12.5 • 10⁻⁶ 1/K.

8. Metal-fixing-material leadthrough according to at least one of the preceding claims,
**characterized in that**
the vitreous or glass-ceramic fixing material (10) has a coefficient of thermal expansion α_{glass} at a temperature up to Tg of the fixing material in the range 4 • 10⁻⁶ 1/K to 10.6 • 10⁻⁶ 1/K.

9. Metal-fixing-material leadthrough according to at least one of the preceding claims,
**characterized in that**
the base body has a coefficient of thermal expansion a_{base body} which is at least 2 • 10⁻⁶ 1/K, preferably 10 • 10⁻⁶ 1/K, higher than the coefficient of thermal expansion α_{glass} of the glass, preferably in the range 11 • 10⁻⁶ 1/K to 18 • 10⁻⁶ 1/K.

10. Metal-fixing-material leadthrough (1) according to at least one of the preceding claims,
**characterized in that**
the at least one metal pin (5) has at least one bending point (50), wherein the metal pin (5) is preferably bent in such a way that there is an axial offset S of the area of the metal pin (5) in the through-opening (4) and of the connection area at its opposite end.

11. Metal-fixing-material leadthrough (1) according to at least one of the preceding claims,
**characterized in that**
at least one further metal pin (6) is electrically conductively connected to the base body (1), in particular by means of a solder connection (7) or a welded connection.

12. Metal-fixing-material leadthrough (1) according to claim 11,
**characterized in that**
the further metal pin (6) connected in an electrically conductive manner to the base body consists, at least in its core region, of a non-stainless steel, in particular of NiFe, and wherein this metal pin (6) is connected to the base body by means of a welded connection, and this metal pin (6) is preferably present in a non-annealed state.

13. Metal-fixing-material leadthrough (1) according to claim 11,
**characterized in that**
the further metal pin (6) has at least one bending point (60), wherein said further metal pin (6) is preferably bent in such a way that there is an axial offset of the portion of the metal pin (6) connected to the base body and of the connection portion at the opposite end thereof.

14. Metal-fixing-material leadthrough (1) according to at least one of the preceding claims,
**characterized in that**
the at least one metal pin (5) vitrified in the through-opening (4) and/or the metal pin (6) electrically conductively connecting to the base body (1) is coated with nickel; wherein the nickel layer is preferably present in areas of the metal pins (5,6) selected from the group including combinations thereof:
- on the vitrified metal pin (5) in an area provided with a gold layer on at least areas of the nickel layer, in particular in the connection area at the end of the metal pin (5),
- on the vitrified metal pin (5) in an area which is in contact with the fixing material,
- on the metal pin (6) electrically conductively connected to the base body in an area provided with a gold layer on at least areas of the nickel layer, in particular in the connection area at the end of the metal pin (5),
- on the metal pin (6) electrically conductively connected to the base body in an area where the metal pin (6) is connected to the base body by means of a metallic solder material (7).

15. Metal-fixing material leadthrough (1) according to at least one of the preceding claims,
**characterized in that**
the at least one metal pin (5) vitrified into the through-opening (4) and/or the further metal pin (6) electrically conductively connected to the base body (1) is coated with gold, wherein the gold layer is preferably present at least in the connection region of the metal pin (5) and/or of the further metal pin (6) electrically conductively connected to the base body, which lies opposite the end of the respective metal pin located in and/or on the base body.

## Revendications

1. Traversée en métal et matériau de fixation pour déclencheur pyrotechnique d'airbag et/ou de tendeur de ceinture de sécurité, avec au moins une goupille métallique (5) qui est incluse dans un matériau de fixation (10) en verre ou en vitrocéramique dans une ouverture de passage (4) d'un corps de base (1), dans laquelle l'au moins une goupille métallique (5) se compose, au moins dans sa partie de coeur, d'un acier spécial selon EN 10020:2000, **caractérisée en ce que** l'acier spécial est un acier spécial ferritique et il est choisi de telle manière que l'au moins une goupille métallique (5) présente, en convertissant au dimensionnement standard d'un diamètre de la goupille métallique de 1,00 ± 0,03 mm et d'une longueur de la goupille métallique de 11,68 ± 0,02 mm, une flexion élastique maximale Wₘₐₓ d'au moins 0,13 mm, de préférence d'au moins 0,15 mm, en particulier d'au moins 0,18 mm, tout particulièrement de 0,24 mm.

2. Traversée en métal et matériau de fixation (1) selon la revendication 1, **caractérisée en ce que** l'acier spécial est choisi de telle manière que, sous une contrainte mécanique comprise entre 3 N et 4 N, il se produise une déviation de l'au moins une goupille métallique (5) d'au maximum 0,21 mm, en particulier une déviation élastique.

3. Traversée en métal et matériau de fixation (1) selon l'une au moins des revendications précédentes, **caractérisée en ce qu'**une contrainte mécanique d'étirement de 0,25 % de l'au moins une goupille métallique (5) correspond à une tension de plus de 450 MPa, de préférence plus de 480 MPa ou plus de 500 MPa, de préférence en particulier de 450 MPa à 700 MPa.

4. Traversée en métal et matériau de fixation (1) selon l'une au moins des revendications précédentes, **caractérisée en ce que** la dureté de l'au moins une goupille métallique (5) dans un état recuit est choisie de telle manière que la force d'extraction de l'au moins une goupille métallique (5) hors du matériau vitreux de l'ouverture de passage (11) soit supérieure à 250 N, en particulier de 250 N à 400 N, de préférence de 300 N à 380 N.

5. Traversée en métal et matériau de fixation (1) selon l'une au moins des revendications précédentes, **caractérisée en ce que** le corps de base (1) se compose d'un métal, en particulier d'acier, d'acier inoxydable, d'acier spécial, de titane, d'un alliage de titane, de magnésium, d'un alliage de magnésium, d'un alliage d'aluminium, d'aluminium ou d'AlSiC et/ou en contient pour l'essentiel.

6. Traversée en métal et matériau de fixation (1) selon la revendication précédente, **caractérisée en ce que** l'acier spécial du corps de base (1) est un acier spécial austénitique.

7. Traversée en métal et matériau de fixation (1) selon l'une au moins des revendications précédentes, **caractérisée en ce que** l'acier spécial de l'au moins une goupille métallique (5) est choisi de telle manière que le coefficient de dilatation thermique α_{goupille_métallique} à une température de 650 °C se situe entre 9 et 15, de préférence entre 11,0 • 10⁻⁶/K et 14,0 • 10⁻⁶/K, de préférence entre 11,5 • 10⁻⁶/K et 14,0 • 10⁻⁶/K, en particulier entre 11 • 10⁻⁶/K et 13,5 • 10⁻⁶/K, tout particulièrement entre 11,5 • 10⁻⁶/K et 12,5 • 10⁻⁶/K.

8. Traversée en métal et matériau de fixation selon l'une au moins des revendications précédentes, **caractérisée en ce que** le matériau de fixation (10) en verre ou en vitrocéramique présente un coefficient de dilatation thermique αᵥₑᵣᵣₑ à une température inférieure ou égale à la Tg du matériau de fixation compris entre 4 • 10⁻⁶/K et 10,6 • 10⁻⁶/K.

9. Traversée en métal et matériau de fixation selon l'une au moins des revendications précédentes, **caractérisée en ce que** le corps de base présente un coefficient de dilatation thermique a_{corps_de_base} qui est supérieur d'au moins 2 • 10⁻⁶/K, de préférence de 10• 10⁻⁶/K, au coefficient de dilatation thermique αᵥₑᵣᵣₑ du verre, de préférence compris entre 11 • 10⁻⁶/K et 18 • 10⁻⁶/K.

10. Traversée en métal et matériau de fixation (1) selon l'une au moins des revendications précédentes, **caractérisée en ce que** l'au moins une goupille métallique (5) présente au moins un point d'inflexion (50), la goupille métallique (5) étant de préférence pliée de façon à obtenir un décalage axial S entre la partie de la goupille métallique (5) située dans l'ouverture de passage (4) et la zone de raccordement à son extrémité opposée.

11. Traversée en métal et matériau de fixation (1) selon l'une au moins des revendications précédentes, **caractérisée en ce qu'**au moins une autre goupille métallique (6) est reliée de façon électriquement conductrice au corps de base (1), en particulier au moyen d'une brasure (7) ou d'une soudure.

12. Traversée en métal et matériau de fixation (1) selon la revendication 11, **caractérisée en ce que** l'autre goupille métallique reliée de façon électriquement conductrice au corps de base se compose d'un acier non affiné, en particulier de NiFe, au moins dans sa partie de coeur, cette goupille métallique (6) étant reliée au corps de base au moyen d'une soudure, et cette goupille métallique (6) se trouve de préférence dans un état non recuit.

13. Traversée en métal et matériau de fixation (1) selon la revendication 11, **caractérisée en ce que** l'autre goupille métallique (6) présente au moins un point d'inflexion (60), cette autre goupille métallique (6) étant de préférence pliée de façon à obtenir un décalage axial entre la partie de la goupille métallique (6) reliée au corps de base et la partie de raccordement à son extrémité opposée.

14. Traversée en métal et matériau de fixation (1) selon l'une au moins des revendications précédentes, **caractérisée en ce que** l'au moins une goupille métallique (5) incluse dans le verre dans l'ouverture de passage (4) et/ou la goupille métallique (6) reliée de façon conductrice électrique au corps de base (1) sont revêtues de nickel, la couche de nickel se trouvant de préférence dans des parties des goupilles métalliques (5, 6) choisies parmi un groupe incluant, y compris des combinaisons :
- sur la goupille métallique (5) incluse dans le verre, dans une partie dotée d'une couche d'or sur au moins des parties de la couche de nickel, en particulier dans la partie de raccordement à l'extrémité de la goupille métallique (5),
- sur la goupille métallique (5) incluse dans le verre, dans une partie en contact avec le matériau de fixation,
- sur la goupille métallique (6) reliée de façon conductrice électrique au corps de base, dans une partie dotée d'une couche d'or sur au moins des parties de la couche de nickel, en particulier dans la partie de raccordement à l'extrémité de la goupille métallique (5),
- sur la goupille métallique (6) reliée de façon conductrice électrique au corps de base, dans une partie dans laquelle la goupille métallique (6) est reliée au corps de base au moyen d'un matériau de brasure métallique (7).

15. Traversée en métal et matériau de fixation (1) selon l'une au moins des revendications précédentes, **caractérisée en ce que** l'au moins une goupille métallique (5) incluse dans le verre dans l'ouverture de passage (4) et/ou la goupille métallique (6) reliée de façon conductrice électrique au corps de base (1) sont revêtues d'or, la couche d'or se trouvant de préférence au moins dans la partie de raccordement de la goupille métallique (5) et/ou de l'autre goupille métallique (6) reliée de façon conductrice électrique au corps de base qui est opposée à l'extrémité de la goupille métallique en question qui se trouve dans et/ou sur le corps de base.
